# EUROPEAN PATENT SPECIFICATION

(11) **EP 2 635 585 B1**
(45) Date of publication and mention of the grant of the patent: **01.11.2017**
(21) Application number: 11788783.6
(22) Date of filing: 04.11.2011
(51) Int. Cl.: C07D 487/22, A61K 39/00

(54) **HIGH-EFFICIENCY CATALYSTS, PREPARATION AND USE THEREOF**
HOCHEFFIZIENTE KATALYSATOREN, IHRE HERSTELLUNG UND VERWENDUNG
CATALYSEURS D'EFFICACITÉ ÉLEVÉE, LEUR SYNTHÈSE ET LEUR UTILISATION

(30) Priority: 05.11.2010 IT MI20102059
(43) Date of publication of application: 11.09.2013
(73) Proprietor: Inkidia S.R.L., 80128 Napoli (IT)
(72) Inventor: PAVONE, Vincenzo, 80121 Napoli (IT); NASTRI, Flavia, 80127 Napoli (IT); MAGLIO, Ornella, 80055 Portici (IT); LOMBARDI, Angelina, 80128 Napoli (IT)
(74) Representative: Banfi, Paolo
(86) International application number: PCT/EP2011/069425
(87) International publication number: WO 2012/059584

(56) References cited:
- LOMBARDI ANGELA ET AL: "Design of a new mimochrome with unique topology.", CHEMISTRY (WEINHEIM AN DER BERGSTRASSE, GERMANY) 21 NOV 2003 LNKD- PUBMED:14639648, vol. 9, no. 22, 21 November 2003 (2003-11-21), pages 5643-5654, XP002633011, ISSN: 0947-6539 cited in the application
- DEL GATTO: "Synthesis and structural characterization of artificial metalloproteins", THESIS UNIVERSITY OF NAPLES,, 1 January 2007 (2007-01-01), pages I-IV, XP007918297,
- P Ringhieri: "Synthetic heme-proteins in biosensors development", Thesis University of Naples, 2 December 2010 (2010-12-02), pages 1-155, XP55016473, Retrieved from the Internet: URL:http://www.fedoa.unina.it/8376/1/Ringh ieri_Paola_23.pdf [retrieved on 2012-01-13]

## Description

### Field of invention

The present invention relates to nitrogen containing macrocycles, porphyrin-like, functionalised with peptide based synthetic compounds, and their preparation and use as catalysts in fine chemistry. They can be used, for example, as catalysts in aqueous or water-alcohol solutions for peroxidation, oxidation, hydroxylation, phenol nitration and inert compound epoxidation reactions, in the control and decontamination of waters and in laboratory diagnostics. They can also be used, for example, in immunohistochemical tests, *in situ* hybridisation, ELISA tests, and Southern, Northern and Western blot tests. The compounds according to the invention can be used "as is" or in covalent association with biomolecules such as antibodies, antigens, enzymes, receptors, nucleic acids, peptides and proteins. The compounds according to the invention have a wide range of applications, because they represent low molecular weight (2000-5000 amu) analogues of natural and mutated haemoproteins, and are therefore proposed as particularly advantageous alternatives to products of expression or extraction. The compounds according to the invention can also be used supported on solid matrices and/or on paramagnetic and non-paramagnetic nanoparticles.

### State of the art

Numerous nitrogen macrocycles of various compositions and with various metal ions inserted in the nitrogen macrocycle are known in the literature. The most promising from the application standpoint are the meso-tetra-aryl substituted porphyrins. ("Metallo-porphyrins in catalytic oxidations" (1994) Sheldon R.A. ed. Dekker, NY; D. Mansuy, C.R. Chimie, 2007, 10, pp 392; Meunier, B. Chem. Rev. 1992, 92, pp. 1411). Despite their application potential, the majority of the nitrogen macrocycles described have not found any industrial application for numerous reasons, including: 1) low solubility in water and biological fluids; 2) low turnover and/or low selectivity in catalytic processes; 3) need to use strong oxidising reagents for activation.

Other porphyrins substituted in the *beta*-pyrrolic and *meso* positions were subsequently developed to improve the catalytic characteristics (see, for example, Bruice T.C. et al. PNAS, 1986, pp4646). However, these compounds also present the severe limitation of solubility in water, and can be used in practice at the aqueous phase/organic phase interface.

Porphyrins substituted in the meso positions with hydrophilic groups (both anionic and cationic types) are water-soluble, but still lack sufficient catalytic efficiency to be useful in fine chemistry industrial applications (Lindsay Smith, J.R. et al. J.Chem.Soc. Chem. Comm 1985, pp410; Bernadou, J. et al. Tetrahedron letters, 1988, pp6615).

New peptide-porphyrin conjugates have been described more recently. They can be grouped on the basis of the number of peptides bonded to the porphyrin, in mono- and di-adducts. For example, microperoxidases are mono-adduct porphyrins obtained from proteolytic digestion of cytochromes c. These compounds are also characterised by two thioether bonds between the cysteines of the peptide component and the vinyl groups of protoporphyrin IX. Depending on the enzyme used in the proteolytic digestion, different microperoxidases are obtained, whose peptide chain is characterised by a different number of aminoacids (Aron, J.; Baldwin, D.A.; Marques, H.M.; Pratt, J.M.; Adams, P.A.J. Inorg. Biochem. 1986, 27, 227). These compounds are commonly called microperoxidases MP8, MP9, MP10 and MP11 (Munro, O.Q.; Marques, H.M. Inorg. Chem. 1996, 35, 3752; Adams, P.A. In: Peroxidases in Chemistry and Biology, 1993, Vol. II, Everse, J., Everse, K.E. & Grisham, M.B., eds, pp. 171-200. CRC Press, Boston, MA, USA; Marques, H.M. Dalton Trans. 2007, 4371).

The peptide sequences of the microperoxidases best characterised to date are set out below:
MP8: H-Cys-Ala-Gln-Cys-His-Thr-Val-Glu-OH (J. Aron, D.A. Baldwin, H.M. Marques, J.M. Pratt, P.A. Adams, J. Inorg. Biochem. 1986, 27, 227; D.A. Baldwin, H.M. Marques, J.M. Pratt, J. Inorg. Biochem. 1986, 27, 245; M.S.A. Hamza, J.M. Pratt, J. Chem. Soc., Dalton Trans. 1994, 1367);
**MP9:** H-Cys-Ala-Gln-Cys-His-Thr-Val-Glu-Lys-OH (Baldwin, D. A.; Mabuya, M. B.; Marques, H. M. S. Afr. J. Chem. 1987, 40, 103); **MP11:** H-Val-Gln-Lys-Cys-Ala-Gln-Cys-His-Thr-Val-Glu-OH (Carraway, A. D.; McCollum, M. G.; Peterson, J. Inorg. Chem. 1996, 35, 6885); **MP'11:** H-Lys-Thr-Arg-Cys-Glu-Leu-Cys-His-Thr-Val-Glu-OH (C. Dallacosta, E. Monzani, L. Casella, ChemBioChem. 5, 2004, 1692); **Ac-MP8:** Ac-Cys-Ala-Gln-Cys-His-Thr-Val-Glu-OH (Munro, O. Q.; Marques, H. M. Inorg. Chem. 1996, 35, 3752; Munro, O. Q.; Marques, H. M. Inorg. Chem. 1996, 35, 3768; Carraway, A. D.; McCollum, M. G.; Peterson, J. Inorg. Chem. 1996, 35, 6885); **bis-Ac-MP11:** Ac-Val-Gln-Lys(Ac)-Cys-Ala-Gln-Cys-His-Thr-Val-Glu-OH (Carraway, A. D.; McCollum, M. G.; Peterson, J. Inorg. Chem. 1996, 35, 6885); **Fmoc-Pro-MP8:** Fmoc-Pro-Cys-Ala-Gln-Cys-His-Thr-Val-Glu-OH (Casella, L.; De Gioia, L.; Frontoso Silvestri, G.; Monzani, E.; Redaelli, C.; Roncone, R.; Santagostini, L. J. Inorg. Biochem. 2000, 79, 31); **Pro-MP8:** H-Pro-Cys-Ala-Gln-Cys-His-Thr-Val-Glu-OH (Casella, L.; De Gioia, L.; Frontoso Silvestri, G.; Monzani, E.; Redaelli, C.; Roncone, R.; Santagostini, L. J. Inorg. Biochem. 2000, 79, 31); **Pro₂-MP8:** H-Pro-Cys-Ala-Gln-Cys-His-Thr-Val-Glu-OH (Casella, L.; De Gioia, L.; Frontoso Silvestri, G.; Monzani, E.; Redaelli, C.; Roncone, R.; Santagostini, L. J. Inorg. Biochem. 2000, 79, 31); **MP9-22 -(FmocAlaPro):** H-Cys-Ala-Gln-Cys-His-Thr-Val-Glu-Lys (Fmoc-Ala-Pro)-OH (Casella, L.; De Gioia, L.; Frontoso Silvestri, G.; Monzani, E.; Redaelli, C.; Roncone, R.; Santagostini, L.J. Inorg. Biochem. 2000, 79, 31).

Microperoxidases are structurally very different from the peptides claimed and, though they present iron pentacoordination, which involves the single histidine residue present in their sequence, they show very modest catalytic activity compared with that of horseradish peroxidase (HRP). Some kinetic parameters in the oxidation of ABTS using H₂O₂ for MP8, MP11 and the HRP enzyme are set out below by way of example.

| **Enzyme** | **pH** | **k_{cat} (s⁻¹)** | **Mw(E) KDa** | **Specific activity (mol g⁻¹ min⁻¹)** |
|---|---|---|---|---|
| MP8 | 7.0 | 0.0026 | 1.51 | 0.104* 10⁻³ |
| MP11 | 7.0 | 0.013 | 1.86 | 0.419* 10⁻³ |
| HRP | 4.6 | 4100 | 44 | 5.569 |
| HRP | 7.0 | 53 | 44 | 0.071 |

Moreover, rapid degradation of the catalyst is observed during the oxidation catalysis of organic substrates by H₂O₂. Another limiting factor in the use of microperoxidases is their low solubility in water. N-acetylated microperoxidases are more soluble in water and have better catalytic activity in the oxidation of organic substrates using H₂O₂ as oxidiser, and are still easily degradable and present modest catalytic activity. Microperoxidases have therefore not yet found an industrial application.

Other examples of peptide-porphyrin mono-adduct conjugates were reported by Casella L. et al. (J. Chem. Soc. Dalton Trans. 1993, 2233) and recently revisited in the review by Nicolis S. et al. (Comptes Rendus Chimie 10, 2007, 380-391). These molecules, as well as being structurally very different from the molecules claimed, present many limitations similar to those of microperoxidases, such as low catalytic activity, and therefore have not yet found an industrial application.

New peptide-porphyrin di-adduct conjugates have been reported by Benson (Benson, D.R.; Hart, B.R.; Zhu, X.; Doughty, M.B. J. Am. Chem. Soc. 1995, 117, 8502; Arnold, P.A.; Benson, D.R.; Brink, D.J.; Hendrich, M.P.; Jas, G.S.; Kennedy, M.L.; Petasis, D.T.; Wang, M. Inorg. Chem. 1997, 36, 5306; Wang, M.; Kennedy, M.L.; Hart, B.R.; Benson, D.R. Chem. Commun. 1997, 883; Williamson, D.A.; Benson, D.R. Chem. Commun. 1998, 961; Liu, D.; Williamson, D.A.; Kennedy, M.L.; Williams, T.D.; Morton, M.M.; Benson, D.R.J. Am. Chem. Soc. 1999, 121, 11798; Liu, D.; Lee, K.-H.; Benson, D.R. Chem. Commun. 1999, 1205; Kennedy, M.L.; Silchenko, S.; Houndonougbo, N.; Gibney, B.R.; Dutton, P.L.; Rodgers, K.R.; Benson, D.R.J. Am. Chem. Soc. 2001, 123, 4635). These compounds are characterised by two equal peptides bonded to the propionyl groups of mesohaem, called PSM (peptide-sandwiched mesohaems): PSM1, Ac-AKEAAHAEAAEAA-NH2; PSM2, Ac-AAEAAEAHAAEKA-NH2; PSM3, Ac-AKEAHAAEAAEAA-NH2; PSM4, Ac-AAEAAEAAHAEKA-NH2; PSM5, Ac-AAHAEAAEAKEAA-NH2; PSM6, Ac-AAEAAEAHAAEKA-NH2; PSM7, Ac-AAEFAEAHAAEKA-NH2; PSM8, Ac-AAEWAEAHAAEKA-NH2.

The presence on these molecules of equal peptides containing a histidine involves iron hexa-coordination; these molecules therefore present very low catalytic activity in reactions involving organic substrate oxidation by H₂O₂, as they lack a distal site for the substrate bond. None of the compounds in this series has yet found an industrial application.

Other di-adduct compounds are those called mimochrome, described by: Nastri F. et al. Chem. Eur. J. 1997, 3, pp340; D'Auria G. et al. Chem. Eur. J. 1997, 3, pp350; Nastri F. et al. J. Biol. Inorg Chem. 1998, 3, pp671; Nastri F. et al. Biopolymers (Peptide Science) 1998, pp5; Lombardi A. et al. Chem Rev. 2001, pp316; Lombardi A. et al. Inorg. Chim. Acta 1998, 275-276, pp301; Lombardi A. et al. Chem. Eur. J. 2003, 9, pp5643; Di Costanzo L. et al. J. Biol. Inorg. Chem. 2004, 9, pp1017; and revisited in the review by Lombardi A. et al. Chem. Rev. 2001, 101, 3165-3189.

Mimochrome I, II and IV (Nastri F. et al. Chem. Eur. J. 1997, 3, pp340; D'Auria G. et al. Chem. Eur. J. 1997, 3, pp350; Lombardi A. et al. Inorg. Chim. Acta 1998, 275-276, pp301; Lombardi A. et al. Chem. Eur. J. 2003, 9, pp5643; Di Costanzo L. et al. J. Biol. Inorg. Chem. 2004, 9, pp1017) are also characterised by bis-histidine iron coordination, and consequently present the same limitations as the PSMs described by Benson, whereas mimochrome III, V and VI (Lombardi A. et al. Chem. Rev. 2001, 101, 3165-3189) present penta-coordination. However, due to the particular aminoacid composition of the two peptide chains, which are of equal length, these latter analogues do not present catalytic activity greater than or comparable to that of natural peroxidases. Some kinetic parameters in ABTS oxidation using H₂O₂ for mimochrome and the HRP enzyme are set out below by way of example.

| **Enzyme** | **pH** | **k_{cat}** | **Mw(E)** | **Specific activity** |
|---|---|---|---|---|
| | | **(s⁻¹)** | **KDa** | **(mol g⁻¹ min⁻¹)** |
| FeIII-Mimochrome I | 7.0 | 0.005 | 2.62 | 1.14 10⁻⁴ |
| FeIII-Mimochrome II | 7.0 | 0.011 | 3.83 | 1.72 10⁻⁴ |
| FeIII-Mimochrome III | 7.0 | 0.15 | 4.03 | 2.23 10⁻³ |
| FeIII-Mimochrome IV | 7.0 | 0.014 | 2.81 | 2.99 10⁻⁴ |
| FeIII-Mimochrome V | 7.0 | 0.16 | 4.00 | 2.40 10⁻³ |
| FeIII-Mimochrome VI | 7.0 | 0.12 | 4.01 | 1.80 10⁻³ |
| HRP | 4.6 | 4100 | 44 | 5.569 |
| HRP | 7.0 | 53 | 44 | 0.071 |

The mimochromes have therefore not yet found an industrial application.

Other more elaborate systems are haemoabzymes (Ricoux R., Raffy Q., Mahy J.P. C.R. Chimie 2007, 10, 684-702), which present the drawback of having a very high molecular weight and being inherently subject to denaturation and degradation. Some kinetic parameters in ABTS oxidation using H₂O₂ for haemoabzymes and HRP enzyme are set out below by way of example.

| **Enzyme** | **pH** | **k_{cat}** | **Mw(E)** | **Specific activity** |
|---|---|---|---|---|
| | | **(s⁻¹)** | **KDa** | **(mol g⁻¹ min⁻¹)** |
| Fe^{III}ToCPP-13G10 | 4.6 | 9.33 | 151 | 3.71 10⁻³ |
| Fe^{III}ToCPP-14H7 | 5.0 | 1.05 | 151 | 0.417 10⁻³ |
| Fe^{III}ToCPP² | 5.0 | 0.85 | 0.878 | 58.1 10⁻³ |
| HRP | 4.6 | 4100 | 44 | 5.569 |
| HRP | 7.0 | 53 | 44 | 0.071 |

Other peptido-porphyrin complex systems were reported by Sasaki T. and Kaiser E.T., JACS 1989, pp380; Akerfeldt K.S. et al. JACS, 1992, pp9656; Arnold, P.A. et al. JACS 1997, pp3181; Sakamoto S. et al. Chem Commun. 1997, pp1221; Kennedy, M. L. et al. JACS 2001, 123, pp 4635 and recently revisited by Reedy, C.J. and Gibney, B.R. (Chem. Rev. 2004, 104, pp. 617). All these compounds present haem centre hexa-coordination, and therefore do not possess any catalytic activity useful for application purposes. In particular, helichrome systems (Sasaki T. and Kaiser E.T., JACS 1989, pp380), tetraphyllin systems (Akerfeldt K.S. et al. JACS, 1992, pp9656) and peptide-mesohaem conjugates (Arnold, P.A. et al. JACS 1997, pp3181; Sakamoto S. et al. Chem Commun. 1997, pp1221) are examples of synthetic peptide-porphyrin conjugates wherein the porphyrin ring has been incorporated covalently into peptide sequences, in order to induce helical structures. The systems developed by Choma C.T. et al. (JACS 1994, pp856) and Robertson D.E. et al. (Nature 1994, pp425) are the first examples of hexacoordinated synthetic haem proteins, consisting of peptide sequences which adopt the "four helix bundle" structural motif, and are able to bind one or more haem groups in their interior in a non-covalent way.

Once again, none of the compounds mentioned above exhibits properties comparable to or greater than those of natural peroxidases.

The structural solutions proposed to date have therefore proved inadequate to meet the following requirements simultaneously: 1) high catalytic efficiency; 2) a high catalytic turnover; 3) adequate solubility in water or water-alcohol solutions; 4) resistance to degradation during catalytic cycles; 5) low molecular weight and high specific activity. Horseradish peroxidase exhibits reduced catalytic activity, at neutrl pH, if compared with its maximal activity at pH=4.6.

Otherwise, natural or mutated enzymes such as peroxidases, cytochromes P450, oxidases and monooxygenases, while meeting the requirements of high catalytic efficiency in reactions requiring activation of H₂O₂, are particularly expensive, liable to denaturation, and consequently may have a limited shelf life, but primarily have rather large molecular dimensions (30,000 - 200,000 Da) which may limit their industrial use. For example, paramagnetic nanoparticles, functionalised with HRP, are known from the literature (Xiaoyan Y. et al., Anal. Biochem. 2009, 393, pp 56). However, the degree of coating is relatively low, involving a limited number of catalytic sites anchored to the nanoparticles, and consequently low catalytic amplification.

Many standard methods of laboratory diagnosis, such as immunohistochemical tests, *in situ* hybridisation, ELISA and blotting, use methods of detecting the desired product which involve incubation of the experimental sample containing the analyte to be detected with a recognition molecule capable of interacting specifically with the analyte. The recognition phenomenon is then quantified by production of a signal, such as a luminescent, colorimetric, fluorimetric, electrochemical or radioactive signal. In many cases, when the analyte is present at low concentrations, the signal needs to be amplified by adding one or more amplification layers to the test. For example, if the recognition element is a primary antibody, a secondary antibody functionalised with an enzyme that catalyses the conversion of a chromogen can be added. For each recognition element, a high quantity of chromogen is therefore produced in a certain time.

Antibodies functionalised with not more than three molecules of horseradish peroxidase are commonly used in ELISA tests. This relatively low degree of substitution is mainly determined by the molecular dimensions of peroxidase (approx. 45000 Da) and therefore, though desirable, antibodies with a higher degree of substitution cannot be obtained. This aspect is particularly relevant in view of the fact that the development of colour depends not only on the number of antibodies bonded in the ELISA test plate, but also on the number of molecules of the enzyme which amplify the molecular recognition. Moreover, small molecules such as peptides, antigens, oligonucleotides, PNA, receptor agonists or antagonists and enzyme inhibitors lose their ability to recognise the target molecules when bonded to large macromolecules such as natural or mutated enzymes.

The natural or mutated enzymes proposed to date for immunohistochemical tests, *in situ* hybridisation, ELISA tests, Southern, Northern and Western blot tests have therefore not proved completely adequate to meet the following requirements simultaneously: 1) very high sensitivity; 2) high stability; 3) versatility and simplicity of use.

Conjugation strategies that can be used without altering the properties of the biomolecules involved are described in the following references: Cross-Linking Reagents Technical Handbook from Pierce Biotechnology; Chemistry of Protein and Nucleic Acid Cross-Linking and Conjugation, First Edition, Shan S. Wong, Ed, 1991, CRC Press, ISBN 9780849358869; Bioconjugate Techniques, Second Edition, Greg T. Hermanson, 2008 Elsevier Inc., ISBN: 978-0-12-370501-3.

The applications of the natural enzyme HRP are described in "Ana M. Azevedo, et al, "Horseradish peroxidase: a valuable tool in biotechnology", Biotechnology Annual Review, Volume 9, 2003, Pages 199-247, doi:10.1016/S1387-2656(03)09003-3.

### Description of the invention

The present invention relates to a new class of peptide-porphyrin conjugates of low molecular weight (2000-5000 Da) wherein the biomimetic properties of high catalytic efficiency are guaranteed by the particular aminoacid composition, which has never previously been described. The compounds according to the invention can be used alone or in covalent association with the desired macromolecules, such as mono- and polyclonal antibodies, antibody fragments, antigens, receptors, receptor agonists and antagonists, biotin, enzymes, enzyme inhibitors, nucleic acids, PNA, peptides and proteins. The compounds according to the invention can also be used supported on solid matrices and/or on paramagnetic and non-paramagnetic nanoparticles.

The described compounds have the following general formula (I): 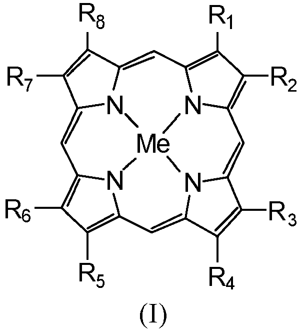 wherein:
the nitrogen atoms of the macrocycle are complexed with the metal ion Me, selected from the group consisting of Fe, Mn and Ru, in any of the possible states of oxidation;
**R1** is a group of general formula (II): 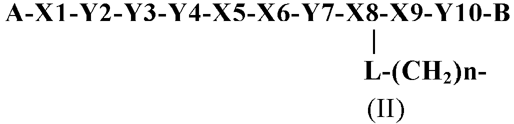 Wherein:
   n = 1, or 2, or 3;
   A is Suc, or Ace, or Ace-Asp, or Ace-Asn, or Ace-Pro, or Ace-Lys-Pro, or Ace-Orn-Pro;
   X1 is an amino acid selected from the group consisting of Glu, Aada, Arg, hArg, Leu, Lys and Orn;
   Y2 is an amino acid selected from the group consisting of Gln, Glu, Lys, Orn;
   Y3 is an amino acid selected from the group consisting of Gln, Glu, Lys, Orn;
   Y4 is Leu;
   X5 is an amino acid selected from the group consisting of His, hCys, Met, 4Taz and 5Taz;
   X6 is an amino acid selected from the group consisting of Ser, Thr, Asn, Gln, aThr, Glu, Lys, Orn;
   Y7 is an amino acid selected from the group consisting of Gln, Glu, Lys, Orn;
   X8 is an amino acid which possesses a functional group on the side chain suitable to form an amide bond selected from the group consisting of Glu, Aada, Orn, Lys, Dap and Dab;
   X9 is an amino acid selected from the group consisting of Glu, Aada, Arg, hArg, Leu, Lys, Orn;
   Y10 is an amino acid selected from the group consisting of Gln, Glu, Lys, Orn;
   B is NH2, or Ile-NH2, or Ile-Thr-NH2, or Ile-Thr-Leu-NH2, or Ile-Thr-Leu-Lys-NH2, or Ile-Thr-Leu-Orn-NH2;
   L is CO if the functional group on the side chain of X8 is an amine, or NH if the functional group on the side chain of X8 is a carboxyl;
**R2** and **R7,** which are equal or different, are H or CH3; one of the substituents **R3, R4, R5, R6** or **R8** has formula Q-(CH₂)s- wherein: s = 1, or 2, or 3 and Q is NH₂CO, or CH₃CONH, or HOOC, or CH₃OOC, or one of the substituents R3, R4, R5, R6 or R8 is a group of general formula (III): 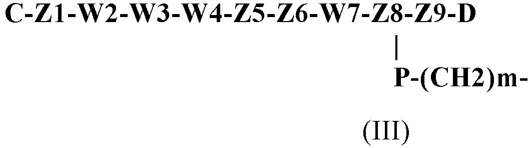 wherein:
   m = 1, or 2, or 3;
   C is Suc, or Ace, or Ace-Asp, or Ace-Asn, or Ace-Pro, or Ace-Lys-Pro or Ace-Orn-Pro;
   Z1 is an amino acid selected from the group consisting of Glu, Aada, Arg, hArg, Leu, Lys and Orn;
   W2 is an amino acid selected from the group consisting of Gln, Glu, Lys, Orn;
   W3 is an amino acid selected from the group consisting of Gln, Glu, Lys, Orn;
   W4 is Leu;
   Z5 is an aminoacid selected from the group consisting of Ser, Gly, Ala and Aib;
   Z6 is an amino acid selected from the group consisting of Gln, Glu, Lys, Orn, Ser, Thr, Asn, aThr;
   W7 is an amino acid selected from the group consisting of Gln, Glu, Lys, Orn;
   Z8 is an amino acid which possesses a functional group on the side chain suitable to form an amide bond selected from the group consisting of Glu, Aada, Orn, Lys, Dap and Dab;
   Z9 is an amino acid selected from the group consisting of Glu, Aada, Orn, Lys, Arg, hArg and Leu;
   D is NH2, or Lys-NH2, or Orn-NH2;
   P is CO if the functional group on the side chain of Z8 is an amine, or NH if the functional group on the side chain of Z8 is a carboxyl;
the remaining substituents **R3, R4, R5, R6** and **R8** not occupied by the group of formula (III), which are equal or different, are selected from H, methyl, ethyl or vinyl.

The table below shows some sequence combinations of the compounds according to general formula (II) and (III) wherein each cell in a row represents possible alternatives in a given position in the sequence:

**General formula (II)**

| | Possible alternatives | | | | | | | | | | | | | | |
|---|---|---|---|---|---|---|---|---|---|---|---|---|---|---|---|
| **A** | Ace Asp | or | Ace Asn | or | Ace Pro | or | Suc | or | Ace | or | Ace Lys Pro | or | Ace Orn Pro | | |
| **X1** | Glu | or | Arg | or | Aada | or | hArg | or | Leu | or | Lys | or | Orn | | |
| **Y2** | Gln | or | Glu | or | Lys | or | Orn | | | | | | | | |
| **Y3** | Gln | or | Glu | or | Lys | or | Orn | | | | | | | | |
| **Y4** | Leu | | | | | | | | | | | | | | |
| **X5** | His | or | 4Taz | or | 5Taz | or | hCys | or | Met | | | | | | |
| **X6** | Ser | or | Thr | or | Asn | or | aThr | or | Gln | or | Glu | or | Lys | or | Orn |
| **Y7** | Gln | or | Glu | or | Lys | or | Orn | | | | | | | | |
| **X8** | Lys | or | Orn | or | Dab | or | Dap | or | Aada | or | Glu | | | | |
| **X9** | Arg | or | Glu | or | hArg | or | Aada | or | Leu | or | Lys | or | Orn | | |
| **Y10** | Lys | or | Orn | or | Gln | or | Glu | | | | | | | | |
| **B** | IleT hrLe uN H2 | or | Ile Thr NH2 | or | Ile NH2 | or | NH2 | or | Ile Thr Leu Lys NH2 | or | Ile Thr Leu Orn NH 2 | | | | |

**General formula (III)**

| | Possible alternatives | | | | | | | | | | | | | | |
|---|---|---|---|---|---|---|---|---|---|---|---|---|---|---|---|
| **C** | Ace Asp | or | Ace Asn | or | Ace Pro | or | Suc | or | Ace | or | Ace Lys Pro | or | Ace Orn Pro | | |
| **Z1** | Glu | or | Arg | or | Aada | or | hArg | or | Leu | or | Lys | or | Orn | | |
| **W 2** | Gln | or | Glu | or | Lys | or | Orn | | | | | | | | |
| **W 3** | Gln | or | Glu | or | Lys | or | Orn | | | | | | | | |
| **W 4** | Leu | | | | | | | | | | | | | | |
| **Z5** | Ser | or | Gly | or | Ala | or | Aib | | | | | | | | |
| **Z6** | Ser | or | Gln | or | Glu | or | Thr | or | aThr | or | Asn | or | Lys | or | Orn |
| **W 7** | Gln | or | Glu | or | Lys | or | Orn | | | | | | | | |
| **Z8** | Lys | or | Orn | or | Dab | or | Dap | or | Aada | or | Glu | | | | |
| **Z9** | Arg | or | Glu | or | Aada | or | hArg | or | Leu | or | Lys | or | Orn | | |
| **D** | NH2 | or | Lys NH2 | or | Orn NH2 | | | | | | | | | | |

The preferred compounds according to the invention are those of general formula (I), wherein:
the nitrogen atoms of the macrocycle are complexed with the metal ion Me, selected from the group consisting of Fe, Mn and Ru, in any of the possible states of oxidation;
**R1** has general formula (II), wherein:
   n = 2, or 3;
   A is Suc, or Ace, or Ace-Asp, or Ace-Asn, or Ace-Pro;
   X1 is an amino acid selected from the group consisting of Glu, Aada, Arg, hArg, Leu;
   Y2 is Gln, or Glu;
   Y3 is Gln, or Glu;
   Y4 is Leu;
   X5 is an amino acid selected from the group consisting of His, hCys, Met, 4Taz and 5Taz;
   X6 is an amino acid selected from the group consisting of Ser, Thr, Asn, Gln, aThr, Glu;
   Y7 is Gln, or Glu;
   X8 is an amino acid which possesses a functional group on the side chain suitable to form an amide bond selected from the group consisting of Glu, Aada, Orn, Lys, Dap and Dab;
   X9 is an aminoacid selected from the group consisting of Glu, Aada, Arg, hArg, Leu;
   Y10 is Lys, or Orn;
   B is NH2, or Ile-NH2, or Ile-Thr-NH2, or Ile-Thr-Leu-NH2;
   L is CO if the functional group on the side chain of X8 is an amine, or NH if the functional group on the side chain of X8 is a carboxyl;
**R2** and **R7,** which are equal or different, are H or CH3;
one of the substituents **R3, R4, R5, R6** or **R8** has formula Q-(CH₂)s- wherein: s = 2, or 3 and Q is NH₂CO, or CH₃CONH, or HOOC, or CH₃OOC, or one of the substituents **R3, R4, R5, R6** or **R8** has general formula (III), wherein:
   m = 2 or 3;
   C is Suc, Ace, Ace-Asp, Ace-Asn, or Ace-Pro;
   Z1 is an amino acid selected from the group consisting of Glu, Aada, Arg, hArg and Leu;
   W2 is Gln, or Glu;
   W3 is Gln, or Glu;
   W4 is Leu;
   Z5 is an amino acid selected from the group consisting of Ser, Gly, Ala and Aib;
   Z6 is an amino acid selected from the group consisting of Gln, Glu, Ser, Thr, Asn, aThr;
   W7 is Gln or Glu;
   Z8 is an amino acid which possesses a functional group on the side chain suitable to form an amide bond selected from the group consisting of Glu, Aada, Orn, Lys, Dap and Dab;
   Z9 is an amino acid selected from the group consisting of Glu, Aada, Orn, hArg and Leu;
   D is NH2;
   P is CO if the functional group on the side chain of Z8 is an amine, or NH if the functional group on the side chain of Z8 is a carboxyl;
the remaining substituents **R3, R4, R5, R6** and **R8** not occupied by the group of formula (III), which are equal or different, are H, methyl, ethyl or vinyl.

The table below shows some sequence combinations of the preferred compounds according to general formula (II) and (III) wherein each cell in a row represents possible alternatives in a given position in the sequence:

**General formula (II)**

| | Possible alternatives | | | | | | | | | | |
|---|---|---|---|---|---|---|---|---|---|---|---|
| **A** | Ace-Asp | or | Ace-Asn | or | Ace-Pro | or | Suc | or | Ace | | |
| **X1** | Glu | or | Arg | or | Aada | or | hArg | or | Leu | | |
| **Y2** | Gln | or | Glu | | | | | | | | |
| **Y3** | Gln | or | Glu | | | | | | | | |
| **Y4** | Leu | | | | | | | | | | |
| **X5** | His | or | 4Taz | or | 5Taz | or | hCys | or | Met | | |
| **X6** | Ser | or | Thr | or | Asn | or | aThr | or | Gln | or | Glu |
| **Y7** | Gln | or | Glu | | | | | | | | |
| **X8** | Lys | or | Orn | or | Dab | or | Dap | or | Aada | or | Glu |
| **X9** | Arg | or | Glu | or | hArg | or | Aada | or | Leu | | |
| **Y10** | Lys | or | Orn | | | | | | | | |
| **B** | Ile-Thr-Leu-NH2 | or | Ile-Thr-NH2 | or | Ile-NH2 | or | NH2 | | | | |

**General formula (III)**

| | Possible alternatives | | | | | | | | | | |
|---|---|---|---|---|---|---|---|---|---|---|---|
| **C** | Ace-Asp | or | Ace-Asn | or | Ace-Pro | or | Suc | or | Ace | | |
| **Z1** | Glu | or | Arg | or | Aada | or | hArg | or | Leu | | |
| **W2** | Gln | or | Glu | | | | | | | | |
| **W3** | Gln | or | Glu | | | | | | | | |
| **W4** | Leu | | | | | | | | | | |
| **Z5** | Ser | or | Gly | or | Ala | or | Aib | | | | |
| **Z6** | Ser | or | Gln | or | Glu | or | Thr | or | aThr | or | Asn |
| **W7** | Gln | or | Glu | | | | | | | | |
| **Z8** | Lys | or | Orn | or | Dab | or | Dap | or | Aada | or | Glu |
| **Z9** | Arg | or | Glu | or | Aada | or | hArg | or | Leu | | |
| **D** | NH2 | | | | | | | | | | |

The more preferred compounds according to the invention are those of general formula (I), wherein:
the nitrogen atoms of the macrocycle are complexed with the metal ion Me, selected from the group consisting of Fe, Mn and Ru, in any of the possible states of oxidation;
**R1** has general formula (II), wherein:
   n=2;
   A is Ace-Asp, or Ace-Asn, or Ace-Pro;
   X1 is selected from the group consisting of Glu, Aada, Arg, hArg, Leu;
   Y2 is Gln or Glu;
   Y3 is Gln or Glu;
   Y4 is Leu;
   X5 is an amino acid selected from the group consisting of His, 4Taz and 5Taz;
   X6 is an amino acid selected from the group consisting of Ser, Thr, Asn, aThr;
   Y7 is Gln or Glu;
   X8 is an amino acid selected from the group consisting of Orn, Lys, Dap and Dab;
   X9 is an amino acid selected from the group consisting of Glu, Aada, Arg, hArg, Leu;
   Y10 is Lys, or Orn;
   B is Ile-Thr-Leu-NH2;
   L is CO;
**R2** and **R7** are CH₃;
**R8** has formula Q-(CH₂)s- wherein: s = 2, and Q is NH₂CO, or CH₃CONH, or HOOC, or CH₃OOC, or **R8** has the general formula (III), wherein:
   m = 2;
   C is Ace-Asp, or Ace-Asn, or Ace-Pro;
   Z1 is an amino acid selected from the group consisting of Glu, Aada, Arg and hArg;
   W2 is Gln, or Glu;
   W3 is Gln, or Glu;
   W4 is Leu;
   Z5 is an amino acid selected from the group consisting of Ser, Gly, Ala and Aib;
   Z6 is an amino acid selected from the group consisting of Gln, Glu, Ser;
   W7 is Gln, or Glu;
   Z8 is an amino acid selected from the group consisting of Orn, Lys, Dap and Dab;
   Z9 is an amino acid selected from the group consisting of Glu, Aada, Arg and hArg;
   D is NH2;
   P is CO;
**R3, R4, R5, R6,** which are equal or different, are H or methyl.

The table below shows some sequence combinations of the more preferred compounds according to general formula (II) and (III) wherein each cell in a row represents possible alternatives in a given position in the sequence:

**General formula (II)**

| | Possible alternatives | | | | | | | | |
|---|---|---|---|---|---|---|---|---|---|
| **A** | Ace-Asp | or | Ace-Asn | or | Ace-Pro | | | | |
| **X1** | Glu | or | Arg | or | Aada | or | hArg | or | Leu |
| **Y2** | Gln | or | Glu | | | | | | |
| **Y3** | Gln | or | Glu | | | | | | |
| **Y4** | Leu | | | | | | | | |
| **X5** | His | or | 4Taz | or | 5Taz | | | | |
| **X6** | Ser | or | Thr | or | Asn | or | aThr | | |
| **Y7** | Gln | or | Glu | | | | | | |
| **X8** | Lys | or | Orn | or | Dab | or | Dap | | |
| **X9** | Arg | or | Glu | or | hArg | or | Aada | or | Leu |
| **Y10** | Lys | or | Orn | | | | | | |
| **B** | Ile-Thr-Leu-NH2 | | | | | | | | |

**General formula (III)**

| | Possible alternatives | | | | | | |
|---|---|---|---|---|---|---|---|
| **C** | Ace-Asp | or | Ace-Asn | or | Ace-Pro | | |
| **Z1** | Glu | or | Arg | or | Aada | or | hArg |
| **W2** | Gln | or | Glu | | | | |
| **W3** | Gln | or | Glu | | | | |
| **W4** | Leu | | | | | | |
| **Z5** | Ser | or | Gly | or | Ala | or | Aib |
| **Z6** | Ser | or | Gln | or | Glu | | |
| **W7** | Gln | or | Glu | | | | |
| **Z8** | Lys | or | Orn | or | Dab | or | Dap |
| **Z9** | Arg | or | Glu | or | Aada | or | hArg |
| **D** | NH2 | | | | | | |

The even more preferred compounds according to the invention are those of general formula (I), wherein:
the nitrogen atoms of the macrocycle are complexed with the metal ion Me, selected from the group consisting of Fe, Mn and Ru, in any of the possible states of oxidation;
**R1** has general formula (II), wherein:
   n=2;
   A is Ace-Asp;
   X1 is Glu, or Arg, or Leu;
   Y2 is Gln, or Glu;
   Y3 is Gln, or Glu;
   Y4 is Leu;
   X5 is His;
   X6 is an amino acid selected from the group consisting of Ser, Thr, Asn, aThr;
   Y7 is Gln, or Glu;
   X8 is Lys;
   X9 is Glu, or Arg, or Leu;
   Y10 is Lys, or Orn;
   B is Ile-Thr-Leu-NH2;
   L is CO;
**R2** and **R7** are CH3;
**R8** has formula Q-(CH₂)s- wherein: s = 2 and Q is NH₂CO, or CH₃CONH, or HOOC, or CH₃OOC, or **R8** has the general formula (III), wherein:
   m = 2;
   C is Ace-Asp;
   Z1 is Glu, or Arg;
   W2 is Gln, or Glu;
   W3 is Gln, or Glu;
   W4 is Leu;
   Z5 is an amino acid selected from the group consisting of Ser, Gly, Ala and Aib;
   Z6 is an amino acid selected from the group consisting of Gln, Glu, Ser;
   W7 is Gln, or Glu, or Aib;
   Z8 is Lys;
   Z9 is Glu, or Arg;
   D is NH2;
   P is CO;
**R3, R4, R5, R6,** which are equal or different, are H or methyl.

The table below shows some sequence combinations of the even more preferred compounds according to general formula (II) and (III) wherein each cell in a row represents possible alternatives in a given position in the sequence:

**General formula (II)**

| | Possible alternatives | | | | | | |
|---|---|---|---|---|---|---|---|
| **A** | Ace-Asp | | | | | | |
| **X1** | Glu | or | Arg | or | Leu | | |
| **Y2** | Gln | or | Glu | | | | |
| **Y3** | Gln | or | Glu | | | | |
| **Y4** | Leu | | | | | | |
| **X5** | His | | | | | | |
| **X6** | Ser | or | Thr | or | Asn | or | aThr |
| **Y7** | Gln | or | Glu | | | | |
| **X8** | Lys | | | | | | |
| **X9** | Arg | or | Glu | or | Leu | | |
| **Y10** | Lys | or | Orn | | | | |
| **B** | Ile-Thr-Leu-NH2 | | | | | | |

**General formula (III)**

| | Possible alternatives | | | | | | | | |
|---|---|---|---|---|---|---|---|---|---|
| **C** | Ace-Asp | | | | | | | | |
| **Z1** | Glu | or | Arg | | | | | | |
| **W2** | Gln | or | Glu | | | | | | |
| **W3** | Gln | or | Glu | | | | | | |
| **W4** | Leu | | | | | | | | |
| **Z5** | Ser | or | Gly | or | Ala | or | Aib | | |
| **Z6** | Ser | or | Gln | or | Glu | | | | |
| **W7** | Gln | or | Glu | | | | | | |
| **Z8** | Lys | | | | | | | | |
| **Z9** | Arg | or | Glu | | | | | | |
| **D** | NH2 | | | | | | | | |

The compounds of general formula (I) can also be used in combination with suitable counterions, provided that they are compatible with the specific applications.

The entirely new, unique properties described hereafter, for compounds with low molecular weight such as the molecules claimed, derive from the structural solutions chosen. Peptide-porphyrin di-adducts with the following structural characteristics are reported and claimed for the first time: a) two peptides of different lengths; one peptide chain contains 10 to 16 aminoacid residues, and the other contains 9 to 12 aminoacid residues; b) two peptides of equal length, namely 10 or 11 or 12 aminoacid residues, never previously described in covalent association with porphyrins. Peptide-porphyrin mono-adducts with the following structural characteristics are also reported and claimed for the first time: a) the peptide chain contains 10 to 16 aminoacid residues; b) the peptide sequence has never previously been described in covalent association with porphyrins.

All these structural solutions surprisingly confer good water-solubility (>mM) on the compounds claimed, even when the various constituents are not mainly hydrophilic. This characteristic is particularly important because it allows the use of the molecules claimed in both aqueous and water-alcohol solutions, thus eliminating the application problems of numerous other modified porphyrins previously reported.

Moreover, unlike the processes reported to date, when the molecules claimed are used as catalysts to activate inert molecules such as H₂O₂, O₂ or NO, the high turnover number and catalytic efficiency is comparable to or greater than that of natural or mutated haemoproteins. This characteristic is an essential requirement for low-cost industrial applications. The compounds described present very high specific activity, as more particularly described in example 13. They are able to convert several kilograms of substrate per minute for each gram of catalyst used.

The specific activity values of some of the compounds claimed in ABTS oxidation using H₂O₂ as oxidising agent are summarised below.

| **Enzyme** | **Specific activity (mol g⁻¹ min⁻¹)** |
|---|---|
| Example 1 | 6.27 |
| Example 4 | 17.86 |
| Example 5 | 11.72 |
| Example 6 | 13.43 |
| Example 7 | 7.98 |
| Example 8 | 6.46 |
| Example 9 | 2.89 |

Moreover, the chemical nature of the compounds according to the invention makes their covalent functionalisation with biomolecules particularly simple, economical and versatile, and above all, the small molecular dimensions provide biological macromolecules, such as mono- and polyclonal antibodies, antibody fragments, antigens, receptors, receptor agonists and antagonists, biotin, enzymes, enzyme inhibitors, nucleic acids, PNA, peptides and proteins, with a very high degree of substitution with the molecules claimed, but without modifying their properties and at the same time increasing the quantity of catalyst present. A chemical, electrochemical or spectroscopic probe can therefore be targeted on a target macromolecule, with the characteristic of amplifying the recognition phenomenon with great efficiency.

Moreover, if the molecules claimed are supported on solid matrices or on nanoparticles, a high degree of coating of the support can be obtained with the methods known from literature. The coating can be up to 10 times greater than that obtained when natural or mutated enzyme systems are used.

The compounds claimed can therefore be used as: 1) catalysts in hydroxylation reactions of aliphatic and aromatic hydrocarbons using clean oxidising agents (H₂O₂, O₂); 2) catalysts in the epoxidation reactions of aliphatic and aromatic olefins using clean oxidising agents (H₂O₂, O₂); 3) catalysts in the oxidation reactions of aliphatic and aromatic hydrocarbons using clean oxidising agents (H₂O₂, O₂); 4) catalysts in the peroxidation reactions of aliphatic and aromatic hydrocarbons using clean oxidising agents (H₂O₂, O₂); 5) phenol nitration catalysts; 6) degradation of pollutants; 7) degradation of lignin; 8) probe to determine the pollutants in water; 9) probe to determinate preservatives in foods; 10) probes to determine the concentration of drugs and toxic products in the body; 11) *in vitro* diagnostics to determine the metabolic pathway of medicaments; 12) determination of the concentration of medicaments and toxic products in biological fluids; 13) immunodiagnostics; 14) immunohistochemical tests; 15) *in situ* hybridisation; 16) in ELISA tests; 17) in Southern, Northern and Western blot tests; 18) cytofluorometry; 19) electrochemical devices.

Moreover, the compounds according to the invention are easy to synthesise and purify, and as they have a low molecular weight, they can be obtained on a large scale at lower costs than haemoproteins obtained by expression or extraction. The proposed synthetic procedure is mainly based on established solid-phase peptide synthesis methods, synthesis with protecting groups characteristic of Fmoc chemistry being preferred. Many of the nitrogen macrocycles required to synthesise the products claimed are commercially available, or can be synthesised with one of the methods described in the literature (Wijesekera T.P. & Dolphin D. in "Metalloporphyrins in catalytic oxidations" 1994, ed. Sheldon R.A., Dekker N.Y.).

The compounds of formula (I) to which this invention relates can be synthesised with the various techniques known from the literature. These techniques include solid-phase peptide synthesis, peptide synthesis in solution, organic chemistry synthesis methods, or any combination thereof. The synthesis scheme chosen will obviously depend on the composition of the particular molecule. Synthetic methods based on appropriate combinations of solid phase techniques and classic methods in solution, which involve low manufacturing costs especially on an industrial scale, are preferably used. In detail, these methods involve:
i) Synthesis in solution of fragments of the peptide chain by successive coupling of suitably activated N-protected aminoacids with an aminoacid or a C-protected peptide chain, with isolation of the intermediates, subsequent selective deprotection of the N- and C-terminal ends of said fragments and their coupling until the desired peptide has been obtained. These stages are followed by selective deprotection of the groups involved in the bond with the nitrogen macrocycle and by their macrocycle condensation. Complete deprotection of the side chains can be performed at that stage, where necessary.
ii) Solid-phase synthesis of the peptide chains from the C-terminal end to the N-terminal end on an insoluble polymer support, selective deprotection of the side chain of residue X8, and detachment from the resin of the peptide protected on the other side chains, where necessary. This is followed by condensation of the peptides with the functional group on the nitrogen macrocycle in the complexed and the non-complexed form with the metal, with one of the methods known from the literature for the formation of an amide bond. This is followed by total deprotection with TFA in the presence of suitable scavengers. Optionally, the metal inserted in the macrocycle contains nitrogen, if not already present.

The following non-limiting examples further illustrate the compounds according to the invention.

**Example 1. Synthesis of 3,7,12,17-tetramethylporphyrin-*2(18)*-N₉^{ε}**-**(Ace-Asp¹-Glu²-Gln³-Gln⁴-Leu⁵-His⁶-Ser⁷-Gln⁸-Lys⁹-Arg¹⁰-Lys¹¹-Ile¹²**-**Thr¹³-Leu¹⁴-NH₂)-*18(2)*-N₉^{ε}-(Ace-Asp¹-Glu²-Gln³-Gln⁴-Leu^{S}-Ser⁶-Ser⁷**-**Gln⁸-Lys⁹-Arg¹⁰-NH₂)-di-propionamide;** compound of general formula (I) in which:
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys (N,epsilon-propionamide); X9 is Arg; Y10 is Lys; B is Ile-Thr-Leu-NH2; L is CO; A is Ace-Asp; R8 is of general formula (III), in which: m = 2; C is Ace-Asp; Z1 is Glu; W2 is Gln; W3 is Gln; W4 is Leu; Z5 is Ser; Z6 is Ser; W7 is Gln; Z8 is Lys (N,epsilon-propionamide); Z9 is Arg; D is NH2; P is CO.

The different steps for the synthesis of 3,7,12,17-tetramethylporphyrin-*2(18)*-N₉^{ε}-(Ace-Asp¹-Glu²-Gln³-Gln⁴-Leu⁵-His⁶-Ser⁷-Gln⁸-Lys⁹-Arg¹⁰-Lys¹¹-Ile¹²-Thr¹³-Leu¹⁴-NH₂)-*18(2)*-N₉^{ε}-(Ace-Asp¹-Glu²-Gln³-Gln⁴-Leu⁵-Ser⁶-Ser⁷-Gln⁸-Lys⁹-Arg¹⁰-NH₂)-di-propionamide are reported in the following.

### Synthesis of the decapeptide (Ace-Asp¹(OtBu)-Glu²(OtBu)-Gln³(Trt)-Gln⁴(Trt)-Leu⁵-Ser⁶(tBu)-Ser⁷(tBu)-Gln⁸(Trt)-Lys⁹-Arg¹⁰(Pbf)-NH₂) (1)

The peptide (1) was synthesized using Fmoc strategy in manual solid-phase peptide synthesis, on Sieber Amide resin. Sieber resin is a 9-Fmoc-amino-xanten-3-yloxy-Merrifield resin (100-200 mesh, 1% 2,2,4,6,7-pentamethyldihydro benzo-furane, substitution level 0.52 mmol g⁻¹), and it is an excellent support for the synthesis of protected peptide amides.

The amino acids were inserted as Fmoc-Asp(OtBu)-OH, Fmoc-Glu(OtBu)-OH, Fmoc-Gln(Trt)-OH, Fmoc-Leu-OH, Fmoc-Ser(tBu)-OH, Fmoc-Arg(Pbf)-OH. The Lys residue in position 9 was inserted as Fmoc-Lys(Mmt)-OH. The Methoxytrityl protecting group (Mmt) is a protecting group that can be removed from the side chain of lysine in mild acid conditions (1% TFA in DCM or AcOH/TFE/DCM 1:2:7 (v/v)); this behaviour allows the selective removal of Mmt group in the presence of other side-chain protecting groups, which require up to 95% TFA for removal. The selective removal of this group allows the coupling of a fully protected peptide, through the free ε-amino group of the Lys 9 side chain, with the porphyrin macrocycle.

The synthesis was carried out on a 0.25 mmol scale. N-αFmoc deprotection was accomplished with a solution of 20% piperidine/DMF (v/v). Two separate treatments, of 3 and 7 minutes, were used for each cycle.

Two coupling steps were performed for each amino acid (45 min coupling time). For the first coupling, 3 equivalents of Fmoc-amino acid, 3 equivalents of PyBop/HOBt, and 6 equivalents of DIEA in N,N-Dimethylformamide (DMF) were used; 2 equivalents of Fmoc-amino acid, 2 equivalents of HATU, and 4 equivalents of DIEA in DMF were employed for the second coupling, instead. Completeness of the reaction was checked after each coupling by the Kaiser test.

After synthesis completion, the N-terminus was acetylated with 4.7% acetic anhydride and 4% pyridine in DMF for 15 min.

Selective removal of Mmt group of Lysine 9 was accomplished by treatment of the peptidyl-resin with AcOH/TFE/DCM 1:2:7 (v/v), in a sintered glass funnel. The resin was shaken for 10 min, and the solvent was removed under vacuum. This step was repeated 15 times. Finally, the resin was washed with isopropanol and DCM. After the clevage of the Mmt group, the fully protected peptide amide was cleaved from the resin by applying a 1% TFA/DCM (volume percentage) solution. The resin was shaken for 2 min, and the filtered solution was collected into an ice-cooled flask containing 5% pyridine/ methanol (volume percentage). This treatment was repeated many times. Finally, the resin was washed with DCM. The filtrates were checked by silica gel TLC in chloroform/methanol/acetic acid 80:18:2 (v/v/v). The fractions containing the desired product were combined and evaporated under reduced pressure up to 5% of the volume.

Ice-cold water was added to the residue and the mixture was cooled on ice to aid precipitation of the protected peptide. The product was filtered, washed several times with fresh water, and dried under vacuum to give the crude C-terminal decapeptide amide (1).

Product homogeinity was ascertained by analytical RP-HPLC, on a C18 column, using a gradient of acetonitrile in 0.1% aqueous TFA, 50% to 95% over 30 min, flow rate 1 mLmin⁻¹). Chromatogram showed a main peak at 23.8 min retention gtime. Peptide identity was checked via ESI-MS spectrometry that confirmed the expected molecular weight (2463 a.m.u.)

The peptide was obtained with a 85% yield.

### Synthesis of tetradecapeptide Ace-Asp¹(OtBu)-Glu²(OtBu)-Gln³(Trt)-Gln⁴(Trt)-Leu⁵-His⁶(Trt)-Ser⁷(tBu)-Gln⁸(Trt)-Lys⁹-Arg¹⁰(Pbf)-Lys¹¹(Boc)-Ile¹²-Thr¹³(tBu)-Leu¹⁴-NH₂)(2)

The synthesis of the tetradecapeptide (2) was performed similarly to decapeptide (1). The amino acids were inserted as Fmoc-Asp(OtBu)-OH, Fmoc-Glu(OtBu)-OH, Fmoc-Gln(Trt)-OH, Fmoc-Leu-OH, Fmoc-Ser(tBu)-OH, Fmoc-Arg(Pbf)-OH, Fmoc-Ile-OH, Fmoc-Thr(tBu)-OH, Fmoc-Lys(Boc)-OH, Fmoc-His(Trt)-OH. The Lys residue in position 9 was inserted as Fmoc-Lys(Mmt)-OH.

Product homogeinity was ascertained by analytical RP-HPLC, on a C18 column, using a gradient of acetonitrile in 0.1% aqueous TFA, 50% to 95% over 30 min, flow rate 1 mLmin⁻¹). Chromatogram showed a main peak at 23.4 min retention time. Peptide identity was checked via ESI-MS spectrometry that confirmed the expected molecular weight (3311 amu).

The peptide was obtained with a 90% yield.

### Synthesis of the peptide-porphyrin intermediate (3): 3,7,12,17-tetramethylporphyrin-18(2)-propionic acid-2(18)-N₉^{ε}-(Ace-Asp¹(OtBu)-Glu²(OtBu)-Gln³(Trt)-Gln⁴(Trt)-Leu⁵-Ser⁶(tBu)-Ser⁷(tBu)-Gln⁸(Trt)-Lys⁹-Arg¹⁰(Pbf)-NH₂) propionamide, monopeptide adduct

This intermediate was synthesized by coupling the decapeptide (1) to the deuteroporphyrin IX (DP-IX) in solution.

Decapeptide (1) (0.100 g, 0.040 mmol) and deuteroporphyrin IX·2HCl (0.028 g, 0.048 mmol) were dissolved in 30 mL of DMF, containing DIEA (0.032 mL, 0.184 mmol). A solution of PyBop (0.025 g, 0.048 mmol), HOBt (0.0075 g, 0.048 mmol), and DIEA (0.017 mL, 0.096 mmol) in DMF (10 mL) was then added dropwise. The reaction mixture was stirred for 3 h at room temperature. The reaction was monitored by analytical HPLC on a C8 column, using a gradient of acetonitrile in 0.1% aqueous TFA, 50% to 90% over 20 min), and by tlc on silica gel (solvent system chloroform/methanol 90:10). The reaction mixture was evaporated under reduced pressure up to 20% of the volume, and precipitated with cold diethylether. The crude product was purified on a silica gel column (5 × 60 cm), with stepwise elution using a chloroform/methanol gradient from 0 to 10% methanol. The product was eluted at 10% methanol, with 48% yield. Analytical RP-HPLC and ESI/MS spectrometry confirmed the purity and identity of the product (2980 amu).

### Synthesis of the final product: 3,7,12,17-tetramethyl porphyrin-2(18)-N₉^{ε}-(Ace-Asp¹-Glu²-Gln³-Gln⁴-Leu⁵-His⁶-Ser⁷-Gln⁸-Lys⁹-Arg¹⁰-Lys¹¹-Ile¹²-Thr¹³-Leu¹⁴-NH₂)-18(2)-N₉^{ε}-(Ace-Asp¹-Glu²-Gln³-Gln⁴-Leu⁵-Ser⁶-Ser⁷-Gln⁸-Lys⁹-Arg¹⁰-NH₂)-di-propionammide (4)

Monopeptide adduct (3) (0.050 g, 0.017 mmol), tetradecapeptide (2) (0.052 g, 0.017 mmol), and DIEA (0.009 mL, 0.051 mmol) were dissolved in 16 mL of 20% TFE (v/v) in DMF. A solution of HATU (0.0065 g, 0.017 mmol) in 1 mL of DMF was then added dropwise, and the reaction was allowed to proceed at room temperature for a total of 2 h. The pH was checked during the reaction time. The reaction progress was followed by analytical HPLC (Vydac C8 column, using a gradient of acetonitrile in 0.1% aqueous TFA, 50% to 90% over 20 min, flow rate 1 mLmin⁻¹), and by tlc (solvent system chloroform/methanol 90:10). The reaction mixture was evaporated under reduced pressure up to 20% of the volume, and precipitated with cold diethylether. The crude product was dried in vacuo. Side chain deprotection was achieved by addition of the cleavage mixture (0.75 g phenol in thioanisole/H₂O/EDT/TFA 0.25/0.5/0.5/8.75, v/v/v/v) (1,2-ethanedithiol: EDT) at 0°C for 2.5 h. This treatment was performed twice. The reaction mixture was concentrated on a rotary evaporator to a volume of approximately 1-2 mL. Extraction of the scavengers and precipitation of the crude product was achieved by addition of cold diethylether. The crude material was then dried in vacuo and purified by preparative RP-HPLC on a C18 column, using a gradient of acetonitrile in 0.1% aqueous TFA, 10% to 80% over 35 min; the pooled fractions containing the desired product were lyophilized, affording 0.025 g (7.2 × 10⁻³ mmol, yield 42%) of the final product. Analytical RP-HPLC indicated that the purified product was > 99% pure, and ESi/MS confirmed the expected molecular weight (3499 amu).

### Synthesis of the Fe3+ complex of the product 3,7,12,17-tetramethyl-porphyrin-2(18)-N₉^{ε}-(Ace-Asp¹-Glu²-Gln³-Gln⁴-Leu⁵-His⁶-Ser⁷-Gln⁸-Lys⁹-Arg¹⁰-Lys¹¹-Ile¹²-Thr¹³-Leu¹⁴-NH₂)-18(2)-N₉^{ε}-(Ace-Asp¹-Glu²-Gln³-Gln⁴-Leu⁵-Ser⁶-Ser⁷-Gln⁸-Lys⁹-Arg¹⁰-NH₂)-di-propionammide (5)

Iron ion was inserted into the macrocycle according to a literature procedure (Buchler JW. in The Porphyrins, Vol. 1 (Ed. D. Dolphin), Academic Press.New York. 1979, pp.389). Fe^{II} acetate (50 molar excess) was added to a solution of the final compound (4) (0.006 g, 1.7 × 10-6 mol, final concentration 1.0 × 10-4 M) acetic acid/TFE 6/4 v/v. The reaction mixture was kept at 40°C for 3 h, refluxing under nitrogen. The reaction was monitored by analytical HPLC. The solvent was then removed under vacuum, and the product was purified to homogeneity by preparative RP-HPLC on a C18 column, using a gradient of acetonitrile in 0.1% aqueous TFA, 10% to 80% over 58.4 min. 0.0032 g (0.90 × 10⁻³ mmol, yield 54%) of pure product were obtained. ESI/MS analysis confirmed the expected molecular weight (3552 amu).

### Example 2. Synthesis of the compound described in example 1 covalently linked to an antibody

The compound described in example 1 was covalently linked to monoclonal murine antibodies anti-Human IgG, referred as IgG in the following, by using a *heterobifunctional linker.* The Sulfo-SMCC (linker) was selected, since this reagent is an amine-to-sulfhydryl crosslinker. *Heterobifunctional linker are* the reagent of choice to obtain high conjugation level, since they do not determine the formation of cross-linking between two identical moieties, such as antibody-antibody. The conjugation protocol was composed of two steps.

### Modification of Lys11 side chain of compound (5) with the reagent Sulfo-SMCC (6)

0.002 g of compound (5) were dissolved in 0.8 mL of 0.1 M phosphate, NaCl 0.15 M at pH=7.2. To this solution, 0.2 mL of an aqueous solution containing 0.0043 g of sulfo-SMCC (molar ratio ≈ 1:20) were added. The reaction mixture was incubated at room temperature for 2 hours, under stirring.

The proceeding of the reaction was followed by LC-MS. When reaction was complete, the reaction mixture was purified using a a PD10 desalting column, using 100 mM phosphate, 150 mM NaCl, pH 7.2, as elution buffer. The fractions containing the desired product (6) were pooled and lyophilized.

### Introduction of sulfhydryl groups into IgG molecules (7)

0.8 mg of N-Succinimidyl S-Acetylthioacetate (SATA), dissolved in 0.010 mL of acetonitrile, were added to a solution of antibody (IgG, 1 mg/mL, ≈ 6.7 10⁻⁶ M) in 0.1 M carbonate buffer, pH 9. The reaction was kept at room temperature for 30 minutes, under stirring.The modified antobodies were purified by using a desalting column, eluted with 0.1 M carbonate buffer, pH 9.

Deacetylation of the SATA-modified antibody was performed by adding 0.100 mL of hydroxylamine stock solution (prepared by dissolving 0.050 g of hydroxylamine hydrochloride in 1.0 ml of 0.1 M carbonate buffer, pH 9) to the SATA-modified antibody solution (NH2OH molar excess is approximately 30-fold over the antibody).

The reaction was kept at room temperature for 2 hours, under stirring. In order to purify the reaction product from the excess hydroxylamine, a desalting column, equilibrated with 100 mM phosphate buffer, 150 mM NaCl, 25 mM EDTA, pH 5 was used.

### Conjugation of the modified antibody (7) with the compound (6) to afford the conjugate product (8)

The final conjugation product (8) was obtained by reacting 0.0025 g of (6) with 0.001 g of modified antibodies(7), in a 0.003 L reaction volume.

The reaction was kept at room temperature for 2 hours, under stirring. The proceeding of the reaction was followed by analytical HPLC using a size-exclusion column equilibrated with 0.1 M phosphate buffer, 3.5 mM SDS, pH6. The conjugation product was purified by gel filtration using a glass column (10 x 150 mm, packed with Sephadex G-100 superfine 10-40). Separation was performed in 0.1 M phosphate buffer, 0.15 M NaCl, pH 7 at a flow rate of 3 mL/h. Conjugation ratio was evaluated by analyzing the absorbance ratio between 280 e 398 nm bands, and was estimated to be 13 (compund (6)/IgG).

**Example 3. Synthesis of the compound 3,7,12,17-tetramethyl-porphyrin-*2(18)*-N₉^{ε}-(Ace-Asp¹-Glu²-Gln³-Gln⁴-Leu⁵-His⁶-Ser⁷-Gln⁸-Lys⁹**-**Arg¹⁰-Lys¹¹-Ile¹²-Thr¹³-Leu¹⁴-NH₂)-*18(2)*-N₉^{ε}-(Ace-Asp¹-Glu²-Gln³-Gln⁴**-**Leu⁵-Ser⁶-Ser⁷-Gln⁸-Lys⁹-Arg¹⁰-NH₂)-di-propionaminide,** of general formula (I) in which:
the nitrogen atoms of the macrocycle are coordinated to the **Ru2+ ion;** R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys(N,epsilon-propionammide); X9 is Arg; Y10 is Lys; B is Ile-Thr-Leu-NH2; L is CO; A is Ace-Asp; R8 is of general formula generale (III), in which: m = 2; C is Ace-Asp; Z1 is Glu; W2 is Gln; W3 is Gln; W4 is Leu; Z5 is Ser; Z6 is Ser; W7 is Gln; Z8 is Lys (N,epsilon-propionamide); Z9 is Arg; D is NH2; P is CO.

The synthesis of this compound started with the insertion of the rutenium ion into the macrocycle (DP-IX). Subsequently, the deca- (compound 1) and the tetradeca-peptide (compound 2), were coupled to the macrocycle as reported in the example 1.

In details, the insertion of rutenium into the porphyrin ring, leading to the preparation of the Ru(II)-CO-DP-IX complex, was carried out using a slightly modified metal carbonyl method.

This method is based on the use of high-boiling aprotic solvents (such as toluene, DMF, dioxane), the triruthenium dodecacarbonyl Ru₃(CO)₁₂ as "metal carrier" and very long reaction time in refluxing conditions. The experimental conditions optimized by us are based on the use of a solution of acetic acid-sodium acetate as solvent (Hartmann M. et al. J.Biol. Inorg. Chem. 1997, 2, pp427). In details, 20.0 mg of DP-IX were dissolved in 6.3 ml of acetic acid solution containing 133.0 mg of sodium acetate. 83 mg of Ru₃(CO)₁₂ (∼4 eq) were added to this solution. The mixture was heated at 85°C, under reflux.

The metallation was monitored by UV-vis spectroscopy and analytical RP-HPLC. The analysis of the mixture after 24 h of reaction time confirmed the insertion of Ru2+ into the macrocycle (80% yield).

The reaction mixture was cooled, and then 50 mL of cold water were addedd to the mixture. The formation of a red-brownish solid containing the desired product, the unreacted DP-IX and the excess of Ru₃(CO)₁₂, was observed. The solid was separated from the solution by centrifugation, and subsequently treated with methanol. Upon methanol addition, unreacted DP-IX and the desired product were dissolved; the unsoluble pellet of Ru₃(CO)₁₂ separated from the solution by centrifugation. The desired product was purified by RP-HPLC chromatography, on a C18 column, 2.2 x 25 cm, using a gradient of acetonitrile in 0.1% aqueous TFA, 20% to 80% over 33 min. The pooled fractions containing the desired product were lyophilized, affording 9.6 mg (15 ×10⁻³ mmol, yield 44%)of pure product.

The homogeneity of the pure product was ascertained by analytical RP-HPLC. The analysis via MALDI mass spectrometry showed the presence of a main peak at m/z 610 amu, corresponding to the Ru(II)-DP-IX molecular ion peak. Infact, during the sample ionization with the MALDI laser source, the dissociation of the CO from the metal ion is observed (Ishii K. et al. Inorg. Chem. 2004, 43, pp 7369). The presence of the CO was confirmed by analysis of the IR spectrum of the product, which showed the v=1989 cm⁻¹ band tipical of a Ru(II)-CO-porphyrin complex.

Coupling of the decapeptide (1) and subsequently of the tetradecapeptide (2) to the Ru(II)-CO-DP-IX were performed as reported in example (1).

The final product was purified by RP-HPLC, and obtained with a 42% yield. The homogeneity and identity were ascertained by LC-MS/ESI analysis (3625 amu).

**Example 4. Synthesis of the compound 3,7,12,17-tetramethylporphyrin-*18(2)*-propionic acid-*2(18)*-N₉^{ε}-(Ace-Asp¹-Glu²**-**Gln³-Gln⁴-Leu⁵-Hiₛ⁶-Ser⁷-Gln⁸-Lys⁹-Arg¹⁰-Lys¹¹-Ile¹²-Thr¹³-Leu¹⁴-NH₂) propionamide,** of general formula (I) in which:
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys(N,epsilon-propionammide); X9 is Arg; Y10 is Lys; B is Ile-Thr-Leu-NH2; L is CO; A is Ace-Asp; R8 is of formula Q-(CH2)m- in which: m = 2, and Q is HOOC.

The synthesis of this compound was carried out using the tetradecapeptide Ace-Asp¹(OtBu)-Glu²(OtBu)-Gln³(Trt)-Gln⁴(Trt)-Leu⁵-His⁶(Trt)-Ser⁷(tBu)-Gln⁸(Trt)-Lys⁹-Arg¹⁰(Pbf)-Lys¹¹(Boc)-Ile¹²-Thr¹³(tBu)-Leu¹⁴-NH₂)(intermediate (2)) described in the example (1) and the DP-IX. The tetradecapeptide was coupled to DP-IX as described in the example 1. After removal of the side chain protecting groups, and insertion of the iron ion into the macrocycle, with the same procedure described in the example (1), the final product was purified by RP-HPLC (48% yield). The homogeneity and identity were ascertained by LC-MS/ESI analysis (2311 amu).

**Example 5. Synthesis of the compound 3,7,12,17-tetramethylporphyrin-*2(18)*-N₉^{ε}-(Ace-Asp¹-Glu²-Gln³-Gln⁴-Leu⁵-His⁶**-**Ser⁷-Gln⁸-Lys⁹-Leu¹⁰-Lys¹¹-Ile¹²-Thr¹³-Leu¹⁴-NH₂)-*18(2)*-N₉^{ε}-(Ace-Asp¹**-**Glu²-Gln³-Gln⁴-Leu⁵-Ser⁶-Ser⁷-Gln⁸-Lys⁹-Arg¹⁰-NH₂)-di-propionamide,** of general formula (I) in which:
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys (N,epsilon-propionamide); X9 is Leu; Y10 is Lys; B is Ile-Thr-Leu-NH2; L is CO; A is Ace-Asp; R8 is of general formula (III), in which: m = 2; C is Ace-Asp; Z1 is Glu; W2 is Gln; W3 is Gln; W4 is Leu; Z5 is Ser; Z6 is Ser; W7 is Gln; Z8 is Lys (N,epsilon-propionamide); Z9 is Arg; D is NH2; P is CO.

The synthesis of this compound was performed using: a) the decapeptide (Ace-Asp¹(OtBu)-Glu²(OtBu)-Gln³(Trt)-Gln⁴(Trt)-Leu⁵-Ser⁶(tBu)-Ser⁷(tBu)-Gln⁸(Trt)-Lys⁹-Arg¹⁰(Pbf)-NH₂) (intermediate 1), synthesized as described in example (1); b) the tetradecapeptide Ace-Asp¹(OtBu)-Glu²(OtBu)-Gln³(Trt)-Gln⁴(Trt)-Leu⁵-His⁶(Trt)-Ser⁷(tBu)-Gln⁸(Trt)-Lys⁹-Leu¹⁰-Lys¹¹(Boc)-Ile¹²-Thr¹³(tBu)-Leu¹⁴-NH₂)(intermediate (2)), containing in position X9 a Leucine instead of the Arginine(Pbf) and synthesized as described in example (1); c) the DP-IX. The coupling between the tetradecapeptide, the decapeptide and the DP-IX was performed as described in the example 1.

After removal of the side chain protecting groups, and insertion of the iron ion into the macrocycle, with the same procedure described in the example (1), the final product was purified by RP-HPLC (25% yield).The homogeneity and identity were ascertained by LC-MS/ESI analysis (3509 amu).

**Example 6. Synthesis of the compound 3,7,12,17-tetramethylporphyrin-*2(18)*-N₉^{ε}-(Ace-Asp¹-Leu²-Gln³-Gln⁴-Leu⁵-His⁶**-**Ser⁷-Gln⁸-Lys⁹-Arg¹⁰-Lys¹¹-Ile¹²-Thr¹³-Leu¹⁴-NH₂)-*18(2)*-N₉^{ε}-(Ace-Asp¹**-**Glu²-Gln³-Gln⁴-Leu⁵-Ser⁶-Ser⁷-Gln⁸-Lys⁹-Arg¹⁰-NH₂)-di-propionamide,** of general formula (I) in which:
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Leu; Y2 is Gln; Y3 is Gln; Y4 is Leu; X5 is His; X6 is Ser; Y7 is Gln; X8 is Lys (N,epsilon-propionammide); X9 is Arg; Y10 is Lys; B is Ile-Thr-Leu-NH2; L is CO; A is Ace-Asp; R8 is of general formula (III), in which: m = 2; C is Ace-Asp; Z1 is Glu; W2 is Gln; W3 is Gln; W4 is Leu; Z5 is Ser; Z6 is Ser; W7 is Gln; Z8 is Lys (N,epsilon-propionammide); Z9 is Arg; D is NH2; P is CO.

The synthesis of this compound was performed using: a) the decapeptide (Ace-Asp¹(OtBu)-Glu²(OtBu)-Gln³(Trt)-Gln⁴(Trt)-Leu⁵-Ser⁶(tBu)-Ser⁷(tBu)-Gln⁸(Trt)-Lys⁹-Arg¹⁰(Pbf)-NH₂) (intermediate 1), synthesized as described in example (1); b) the tetradecapeptide Ace-Asp¹(OtBu)-Leu²-Gln³(Trt)-Gln⁴(Trt)-Leu⁵-His⁶(Trt)-Ser⁷(tBu)-Gln⁸(Trt)-Lys⁹-Arg¹⁰(Pbf)-Lys¹¹(Boc)-I1e¹²-Thr¹³(tBu)-Leu¹⁴-NH₂) (intermediate (2)), containing in position X1 a Leucine instead of the Glu(OtBu), and synthesized as described in example (1); c) the DP-IX. The coupling between the tetradecapeptide, the decapeptide and the DP-IX was performed as described in the example 1.

After removal of the side chain protecting groups, and insertion of the iron ion into the macrocycle, with the same procedure described in the example (1), the final product was purified by RP-HPLC (27% yield).The homogeneity and identity were ascertained by LC-MS/ESI analysis (3536 amu).

**Example 7. Synthesis of the compound 3,7,12,17-tetramethylporphyrin-2(18)-N₉^{ε}-(Ace-Asp¹-Glu²-Gln³-Gln⁴-Leu⁵-His⁶**-**Ser⁷-Gln⁸-Lys⁹-Arg¹⁰-Lys¹¹-Ile¹²-Thr¹³-Leu¹⁴-NH₂)-*18(2)*-N₉^{ε}-(Ace-Asp¹**-**Glu²-Gln³-Gln⁴-Leu⁵-Gly⁶-Ser⁷-Gln⁸-Lys⁹-Arg¹⁰-NH₂)-di-propionamide,** of general formula (I) in which
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys (N,epsilon-propionammide); X9 is Arg; Y10 is Lys; B is Ile-Thr-Leu-NH2; L is CO; A is Ace-Asp; R8 is of general formula (III), in which: m = 2; C is Ace-Asp; Z1 is Glu; W2 is Gln; W3 is Gln; W4 is Leu; Z5 is Gly; Z6 is Ser; W7 is Gln; Z8 is Lys (N,epsilon-propionamide); Z9 is Arg; D is NH2; P is CO.

The synthesis of this compound was performed using: a) the decapeptide (Ace-Asp¹(OtBu)-Glu²(OtBu)-Gln³(Trt)-Gln⁴(Trt)-Leu⁵-Gly⁶-Ser⁷(tBu)-Gln⁸(Trt)-Lys⁹-Arg¹⁰(Pbf)-NH₂) (intermediate 1), synthesized as described in example (1) and containing in position Z5 a glicine instead of the serine(tBu); b) the tetradecapeptide Ace-Asp¹(OtBu)-Glu²(OtBu)-Gln³(Trt)-Gln⁴(Trt)-Leu⁵-His⁶(Trt)-Ser⁷(tBu)-Gln⁸(Trt)-Lys⁹-Arg¹⁰(Pbf)-Lys¹¹(Boc)-Ile¹²-Thr¹³(tBu)-Leu¹⁴-NH₂)(intermediate (2)), synthesized as described in example (1); c) the DP-IX.

The coupling between the tetradecapeptide, the decapeptide and the DP-IX was performed as described in the example 1. After removal of the side chain protecting groups, and insertion of the iron ion into the macrocycle, with the same procedure described in the example (1), the final product was purified by RP-HPLC (28% yield).The homogeneity and identity were ascertained by LC-MS/ESI analysis (3522 amu).

**Example 8. Synthesis of the compound 3,7,12,17-tetramethylporphyrin-*2(18)*-N₉^{ε}-(Ace-Asp¹-Glu²-Gln³-Gln⁴-Leu⁵-His⁶**-**Ser⁷-Gln⁸-Lys⁹-Arg¹⁰-Lys¹¹-Ile¹²-Thr¹³-Leu¹⁴-NH₂)-*18(2)*-N₉^{ε}-(Ace-Asp¹**-**Leu²-Gln³-Gln⁴-Leu⁵-Ser⁶-Ser⁷-Gln⁸-Lys⁹-Arg¹⁰-NH₂)-di-propionamide,** of general formula (I) in which:
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys (N,epsilon-propionammide); X9 is Leu; Y10 is Lys; B is Ile-Thr-Leu-NH2; L is CO; A is Ace-Asp; R8 is of general formula (III), in which: m = 2; C is Ace-Asp; Z1 is Leu; W2 is Gln; W3 is Gln; W4 is Leu; Z5 is Ser; Z6 is Ser; W7 is Gln; Z8 is Lys (N,epsilon-propionamide); Z9 is Arg; D is NH2; P is CO.

The synthesis of this compound was performed using: a) the decapeptide (Ace-Asp¹(OtBu)-Leu²-Gln³(Trt)-Gln⁴(Trt)-Leu⁵-Gly⁶-Ser⁷(tBu)-Gln⁸(Trt)-Lys⁹-Arg¹⁰(Pbf)-NH₂) (intermediate 1), synthesized as described in example (1) and containing in position Z1 a Leucine instead of the Glu(OtBu); b) the tetradecapeptide Ace-Asp¹(OtBu)-Glu²(OtBu)-Gln³(Trt)-Gln⁴(Trt)-Leu⁵-His⁶(Trt)-Ser⁷(tBu)-Gln⁸(Trt)-Lys⁹-Arg¹⁰(Pbf)-Lys¹¹(Boc)-Ile¹²-Thr¹³(tBu)-Leu¹⁴-NH₂)(intermediate (2)), synthesized as described in the example (1); c) the DP-IX.

The coupling between the tetradecapeptide, the decapeptide and the DP-IX was performed as described in the example 1. After removal of the side chain protecting groups, and insertion of the iron ion into the macrocycle, with the same procedure described in the example (1), the final product was purified by RP-HPLC (30% yield).The homogeneity and identity were ascertained by LC-MS/ESI analysis (3536 amu).

**Example 9. Synthesis of the compound 3,7,12,17-tetramethylporphyrin-*2(18)*-N₉^{ε}-(Ace-Asp¹-Glu²-Gln³-Gln⁴-Leu⁵-His⁶**-**Ser⁷-Gln⁸-Lys⁹-Arg¹⁰-Lys¹¹-Ile¹²-Thr¹³-Leu¹⁴-NH₂)-*18(2)*-N₉^{ε}-(Ace-Asp¹**-**Glu²-Gln³-Gln⁴-Leu⁵-Ser⁶-Ser⁷-Gln⁸-Lys⁹-Leu¹⁰-NH₂)-di-propionamide, of** general formula (I) in which:
the nitrogen atoms of the macrocycle are coordinated to the Fe3+ ion; R2 and R7 are CH3; R4 and R6 are H; R3 and R5 are CH3; R1 is of general formula (II), in which: n = 2; A is Ace-Asp, X1 is Glu; Y2 is Gln; Y3 is Gln; Y4 is Leu; X5 is His; X6 is Ser; Y7 is Gln; X8 is Lys (N,epsilon-propionammide); X9 is Leu; Y10 is Lys; B is Ile-Thr-Leu-NH2; L is CO; A is Ace-Asp; R8 is of general formula (III), in which: m = 2; C is Ace-Asp; Z1 is Glu; W2 is Gln; W3 is Gln; W4 is Leu; Z5 is Ser; Z6 is Ser; W7 is Gln; Z8 is Lys (N,epsilon-propionamide); Z9 is Leu; D is NH2; P is CO.

The synthesis of this compound was performed using: a) the decapeptide (Ace-Asp¹(OtBu)-Leu²-Gln³(Trt)-Gln⁴(Trt)-Leu⁵-Gly⁶-Ser⁷(tBu)-Gln⁸(Trt)-Lys⁹-Leu¹⁰-NH₂) (intermediate 1), synthesized as described in example (1) and containing in position Z9 a Leucine instead of the Arg(Pbf); b) the tetradecapeptide Ace-Asp¹(OtBu)-Glu²(OtBu)-Gln³(Trt)-Gln⁴(Trt)-Leu⁵-His⁶(Trt)-Ser⁷(tBu)-Gln⁸(Trt)-Lys⁹-Arg¹⁰(Pbf)-Lys¹¹(Boc)-Ile¹²-Thr¹³(tBu)-Leu¹⁴-NH₂) (intermediate (2)), synthesized as described in the example (1); c) the DP-IX.

The coupling between the tetradecapeptide, the decapeptide and the DP-IX was performed as described in the example 1. After removal of the side chain protecting groups, and insertion of the iron ion into the macrocycle, with the same procedure described in the example (1), the final product was purified by RP-HPLC (28% yield).The homogeneity and identity were ascertained by LC-MS/ESI analysis (3509 amu).

### Example 10. Peroxidase activity of the compound described in example 1

Here is described the peroxidase activity of the compound Fe(III)-3,7,12,17-tetramethylporphyrin-*2(18)*-N₉^{ε}-(Ace-Asp¹-Glu²-Gln³-Gln⁴-Leu⁵-His⁶-Ser⁷-Gln⁸-Lys⁹-Arg¹⁰-Lys¹¹-Ile¹²-Thr¹³-Leu¹⁴-NH₂)-*18(2)*-N₉^{ε}-(Ace-Asp¹-Glu²-Gln³-Gln⁴-Leu⁵-Ser⁶-Ser⁷-Gln⁸-Lys⁹-Arg¹⁰-NH₂)-di-propionammide, whose synthesis was described in example 1, by using H₂O₂ and the following secondary substrates: ABTS and guaiacol.

The peroxidase activity of the compound on the secondary substrate ABTS was evaluated by following the formation of the ABTS^{+•} radical cation, in the presence of H₂O₂.

The reaction was followed by using spectrophotometry, measuring the appearance of the products in the reaction medium. The formation of ABTS^{+•} cation radical was followed at 660 nm (λₘₐₓ (ε) = 660 nm (1.40 × 10⁴ M⁻¹cm⁻¹)). The reaction was performed in 50% TFE (v/v) 100 mM phosphate buffer, pH 6.5, using a 2.0 × 10⁻⁷ M catalyst concentration.

Kinetic parameters of the compound were determined by varying the H₂O₂ concentration using fixed concentrations of the reducing substrates, and *vice versa.*

In more details, in the experiments performed at various H₂O₂ concentrations (in the range 0.01 ÷ 200 mM) the ABTS concentration was kept constant at 0.1 mM. In the experiments performed at various ABTS concentrations (in the range 0.005 ÷ 0.1 mM) the H₂O₂ concentration was 50 mM.

Experimental data were fitted using a two-substrate Michaelis-Menten kinetic model, and the following kinetic parameters were obtained: Kₘ^{AH}₂ 8.4 ± 0.2 10⁻² mM and k_{cat} 370.9 ± 14 s⁻¹.

The peroxidase activity on the guaiacol substrate was evaluated by following the formation of guaiacol oxidation product, tetraguaiacol. Changes in the absorbance at 470 nm were measured, considering a ε*₄₇₀* = 2.66 × 10⁴ M⁻¹cm⁻¹.

In details, in the experiments performed at various H₂O₂ concentrations (in the range 1 ÷ 40 mM), the guaiacol concentration was kept constant at 0.1 mM. In the experiments performed at various guaiacol concentrations, in the range 0.0025 ÷ 0.07 mM, the H₂O₂ concentration was 10 mM.

Experimental data were fitted using a two-substrate Michaelis-Menten kinetic model, and the following kinetic parameters were obtained: Kₘ^{AH}₂ 9.2 ± 0.4 10⁻³ mM and k_{cat} 8.0 ± 0.1 s⁻¹.

The specific activity at pH=6.5 for ABTS oxidation is 104 mmol g⁻¹ s⁻¹. This value is similar to that of horseradish peroxidase (93 mmol g⁻¹ s⁻¹, pH 4.6). For guaiacol oxidation the specific activity is two times higher respect to that of horseradish peroxidase.

### Example 11. Phenol nitration

For the compound Fe(III)-3,7,12,17-tetramethylporphyrin-*2(18)*-N₉^{ε}-(Ace-Asp¹-Glu²-Gln³-Gln⁴-Leu⁵-His⁶-Ser⁷-Gln⁸-Lys⁹-Arg¹⁰-Lys¹¹-I1e¹²-Thr¹³-Leu¹⁴-NH₂)-*18(2)*-N₉^{ε}-(Ace-Asp¹-Glu²-Gln³-Gln⁴-Leu⁵-Ser⁶-Ser⁷-Gln⁸-Lys⁹-Arg¹⁰-NH₂)-di-propionammide, whose synthesis is described in example 1, the ability to catalyze phenol nitration was evaluated. The reaction mixtures were analyzed by analytical HPLC on a Phenomenex Gemini C18 column (150 × 4.6 mm, 5 µm), eluted with a H₂O/0.1% TFA (A) and CH₃CN/0.1% TFA (B) linear gradient from 10% to 90% B over 20 min, at 1 mL/min flow rate. The concentrations of the starting material and products were determined from calibration curves constructed using commercial samples and 4-cyanophenol as internal standard. Standard incubation for phenol nitration were performed at 1 mM phenol in the presence of 0.2 µM catalyst, 20 mM NaNO₂ and 3 mM H₂O₂ in phosphate buffer, pH 6.5, 50% TFE (v/v).

The reactions were performed at room temperature with an incubation time of 40 min. Analysis of the reaction mixture through RP-HPLC showed the formation of both 4- and 2-nitrophenol. The yield of 4- and 2-nitrophenol at 40 min reaction time increased by increasing H₂O₂ NO₂⁻ and phenol concentrations. The maximum yield of nitrophenols (4- and 2-) was obtained at a substrate and oxidant concentrations of 1.0 mM, and at 40 mM NO₂⁻ concentration, respectively.

In these conditions, the total yield of nitrophenols was about 14.8%. The total yield of nitrophenols in the presence of lactoperoxidase is only fourfold higher.

### Example 12. Catalytic activity of the compound described in the example 2.

As an example, in the following is described the peroxidase activity of the compound of formula Fe(III)-3,7,12,17-tetramethylporphyrin-*2(18)*-N₉^{ε}-(Ace-Asp¹-Glu²-Gln³-Gln⁴-Leu⁵-His⁶-Ser⁷-Gln⁸-Lys⁹-Arg¹⁰-Lys¹¹-I1e¹²-Thr¹³-Leu¹⁴-NH₂)-*18(2)*-N₉^{ε}-(Ace-Asp¹-Glu²-Gln³-Gln⁴-Leu⁵-Ser⁶-Ser⁷-Gln⁸-Lys⁹-Arg¹⁰-NH₂)-di-propionamide, covalently linked to an antibody, synthesized as reported in the example 2, using H₂O₂ as oxidizing agent and ABTS as reducing substrate.

The experiments were performed in 50% TFE (v/v) 100 mM phosphate buffer, pH 6.5. As described in the example 10, the catalytic process was evaluated by following the formation of the ABTS^{+•} radical cation at 660 nm (ε=14700 M⁻¹ cm⁻¹). Kinetic parameters of the compound were determined by varying the H₂O₂ concentration using fixed concentrations of the reducing substrates, and *vice versa.*

In more details, in the first experiment the ABTS and the conjugate concentrations were kept constant at 0.1 mM and 2 10⁻⁷ M, respectively, and the H₂O₂ concentration was varied in the range 10 - 100 mM. In the second experiment, the H₂O₂ and the conjugate concentrations were fixed at 50 mM and 2 10⁻⁷ M, respectively, and the ABTS concentration was varied in the range 0.005 ÷ 0.1 mM.

Experimental data were fitted using a two-substrate Michaelis-Menten kinetic model, and the following kinetic parameters were obtained: Kₘ^{AH}₂ 0.123 mM mM and k_{cat} 91 s⁻¹.

### Example 13. Comparison of the catalytic activities

The catalytic activities of the compounds described in the examples 4, 5, 6, 7, 8 e 9 were determined similarly to that described in the example 10.

A comparison of the catalytic activities toward ABTS oxidation in the presence of H₂O₂ of the compounds described in the examples 1, 4, 5, 6, 7, 8 e 9 with those of native and recombinant peroxidases, and other analogues, is reported in the following.

**Catalytic activity for ABTS oxidation with H₂O₂**

| **Enzyme** | **pH** | **Kₘ^{H}₂^{O}₂** | **Kₘ^{AH}₂** | **k_{cat}** | **Mw(E)** | **Specific activity** |
|---|---|---|---|---|---|---|
| | | **(mM)** | **(mM)** | **(s⁻¹)** | **KDa** | **(mol g⁻¹ min⁻¹)** |
| Example 1 | 6.5 | 44 | 0.084 | 371 | 3.55 | 6.269 |
| Example 4 | 6.5 | 80 | 0.130 | 688 | 2.31 | 17.860 |
| Example 5 | 6.5 | 54 | 0.038 | 685 | 3.51 | 11.716 |
| Example 6 | 6.5 | 31 | 0.050 | 785 | 3.54 | 13.430 |
| Example 7 | 6.5 | 46 | 0.125 | 468 | 3.52 | 7.976 |
| Example 8 | 6.5 | 96 | 0.115 | 381 | 3.54 | 6.463 |
| Example 9 | 6.5 | 20 | 0.029 | 169 | 3.51 | 2.890 |
| HRP | 4.6 | - | 0.800 | 4100 | 44.2 | 5.569 |
| HRP | 7.0 | 0.0115 | 5.100 | 53 | 44.2 | 0.071 |
| Mimochrome I | 7.0 | - | - | 0.005 | 2.62 | 1.14 10⁻⁴ |
| Mimochrome II | 7.0 | - | - | 0.011 | 3.83 | 1.72 10⁻⁴ |
| Mimochrome III | 7.0 | - | - | 0.15 | 4.03 | 2.23 10⁻³ |
| Mimochrome IV | 7.0 | - | - | 0.014 | 2.81 | 2.99 10⁻⁴ |
| Mimochrome V | 7.0 | - | - | 0.16 | 4.00 | 2.40 10⁻³ |
| Mimochrome VI | 7.0 | - | - | 0.12 | 4.01 | 1.80 10⁻³ |
| MP8* | 7.0 | - | - | 0.0026 | 1.51 | 0.104 10⁻³ |
| MP11* | 7.0 | - | - | 0.013 | 1.86 | 0.419 10⁻³ |
| ToCPP-13G10 | 4.6 | 16 | - | 9.33 | 151 | 3.71 10⁻³ |
| ToCPP-14H7 | 5.0 | 9 | - | 1.05 | 151 | 0.417 10⁻³ |
| ToCPP2 | 5.0 | 42 | - | 0.85 | 0.878 | 58.1 10⁻³ |

The catalytic activity expressed in terms of specific activity (mol g⁻¹ min⁻¹), that corresponds to the moles of substrate converted per minutes per gram of catalyst, are also listed. For all the compounds described in the example, the specific activities are up to 200-fold higher than native peroxidase at neutral pH and higher or comparable in the condition of maximum activity for HRP (pH=4.6). The specific activity is up to 100,000 fold higher than that of compounds in the prior art (MP8, MP11, ToCPP-13G10, ToCPP-14H7), and also higher than that of the Mimochrome family.

### List of abbreviations

For the nomenclature and abbreviations of the aminoacids, see the recommendations of the IUPAC-IUB Joint Commission on Biochemical Nomenclature (Eur. J. Biochem. 1984, 138, pp 9); "side chain" means any chain on the alpha carbon of an alpha amino acid; the amino acids are in the L configuration unless otherwise specified. The other abbreviations used are:
Suc = succinyl, Ace = acetyl, Asp = aspartic acid, Asn = asparagine, Pro = proline, Lys = lysine, Orn = ornithine, Glu = glutamic acid, Aada = alpha-aminoadipic acid, Arg = arginine, hArg = homoarginine, Leu = leucine, Gln = glutamine, His = histidine, hCys = homocysteine, Met = methionine, 4TAZ = beta-(4-thiazolyl)-alanine, 5TAZ = beta-(5-thiazolyl)-alanine, Ser = serine, Thr = threonine, aThr = allo-threonine, Dap = 2,3 diaminopropionic acid, Dab = 2,4 diaminobutyric acid, Ile = isoleucine, Gly = glycine, Ala= alanine, Aib= alpha-aminoisobutyric acid, Fmoc = fluorenylmethoxycarbonyl, TFA = trifluoroacetic acid, tBu = tert-butyl, Trt = trityl, PBF = 2,2,4,6,7-pentamethyldihydrobenzofuran, DVB = divinylbenzene, Mmt = methoxytrityl, DMF = dimethylformamide, PyBop = benzotriazole-1-yl-oxy-tris-pyrrolidino-phosphonium hexafluorophosphate, HOBt = hydroxybenzotriazole, IDEA = diisopropylethylamine, HATU = 2-(7-aza-1H-benzotriazole-1-yl)-1,1,3,3-tetramethyluronium hexafluorophosphate, TFE = trifluoroethanol, DCM = dichloromethane, Boc = tert-butyloxycarbonyl, sulpho-SMCC = sulphosuccinimidyl-4-(N-maleimidomethyl) cyclohexan-1-carboxylate, EDTA = ethylenediamine tetraacetate, SDS = sodium dodecylsulphate, ABTS = 2,2'-azino-bis-3-ethyl-benzothiazine-6-sulphonic acid, DP-IX = deuteroporphyrin IX.

## Claims

1. A compound of general formula (I) 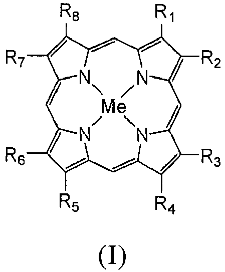 wherein:
the nitrogen atoms of the nitrogen-containing macrocycle are complexed with a metal ion Me, selected from the group consisting of Fe, Mn, Ru, in any permitted oxidation state;
R1 is a group of general formula (II): 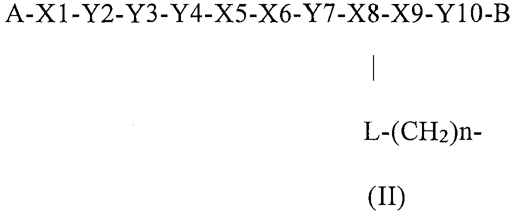
wherein n = 2;
A is selected from the group consisting of Ace-Asp, Ace-Asn and Ace-Pro;
X1 is selected from the group consisting of Glu, Aada, Arg, hArg, and Leu;
Y2 is Gln or Glu;
Y3 is Gln or Glu;
Y4 is Leu;
X5 is selected from the group consisting of His, 4Taz and 5Taz;
X6 is selected from the group consisting of Ser, Thr, Asn and aThr;
Y7 is Gln or Glu;
X8 is selected from the group consisting of Orn, Lys, Dap and Dab;
X9 is selected from the group consisting of Glu, Aada, Arg, hArg, and Leu;
Y10 is Lys or Orn;
B is Ile-Thr-Leu-NH2;
L is CO;
R2 and R7 are CH3;
R8 has formula Q-(CH2)s- wherein s = 2 and Q is selected from the group consisting of NH2CO-, CH3CONH-, HOOC- and CH3OOC- or R8 has general formula (III): 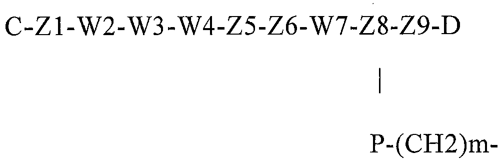
wherein m = 2;
C is Ace-Asp, Ace-Asn or Ace-Pro;
Z1 is selected from the group consisting of Glu, Aada, Arg and hArg;
W2 is Gln or Glu;
W3 is Gln or Glu;
W4 is Leu;
Z5 is selected from the group consisting of Ser, Gly, Ala and Aib;
Z6 is selected from the group consisting of Gln, Glu and Ser;
W7 is Gln or Glu;
Z8 is selected from the group consisting of Orn, Lys, Dap and Dab;
Z9 is an amino acid selected from the group consisting of Glu, Aada, Arg and hArg;
D is NH2;
P is CO;
R3, R4, R5, R6, independently of one another are H or methyl.

2. A compound according to claim 1 wherein:
the nitrogen atoms of the macrocycle are complexed with a metal ion Me, selected from the group consisting of Fe, Mn and Ru, in any permitted oxidation state;
R1 is of general formula (II), wherein:
A is Ace-Asp;
X1 is Glu or Arg or Leu;
Y2 is Gln or Glu;
Y3 is Gln or Glu;
Y4 is Leu;
X5 is His;
X6 is selected from the group consisting of Ser, Thr, Asn and aThr;
Y7 is Gln or Glu;
X8 is Lys;
X9 is Glu or Arg, or Leu;
Y10 is Lys or Orn;
B is Ile-Thr-Leu-NH2;
L is CO;
R2 and R7 are CH3;
R8 has formula Q-(CH2)s- wherein s = 2 and Q is selected from the group consisting of NH2CO-, CH3CONH-, HOOC- and CH3OOC-, or R8 has general formula (III), wherein:
C is Ace-Asp;
Z1 is Glu or Arg;
W2 is Gln or Glu;
W3 is Gln or Glu;
W4 is Leu;
Z5 is selected from the group consisting of Ser, Gly, Ala and Aib;
Z6 is selected from the group consisting of Gln, Glu and Ser;
W7 is Gln or Glu;
Z8 is Lys;
Z9 is Glu or Arg;
D is NH2;
P is CO;
R3, R4, R5, R6, independently of one another are H or methyl.

3. A compound according to claims 1-2, which is covalently bound to a antigen; antibody or fragment thereof; enzyme.

4. A compound according to claims 1-3, which is supported on a solid matrix; nanoparticles or electrode.

5. The use of a compound according to claims 1-4 as a catalyst in the following reactions: epoxidation, oxidation, hydroxylation, nitration or peroxidation.

6. The use of a compound according to claims 1-4 in: immunodiagnostic kit; immunohistochemistry; in-situ hybridization kit; ELISA; Southern blotting; Northern blotting; Western blotting; or in cytofluorimetry.

7. The use of a compound according to claims 1-4
- for the degradation of pollutants.

## Patentansprüche

1. Eine Verbindung der allgemeinen Formel (I) 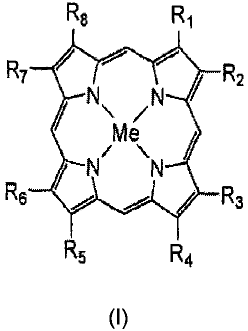 worin:
die Stickstoffatome des Stickstoff enthaltenden Makrozyklus mit einem Metallion Me ausgewählt aus der Gruppe bestehend aus Fe, Mn, Ru, in jeder erlaubten Oxidationsstufe, komplexiert sind;
R1 eine Gruppe der allgemeinen Formel (II): 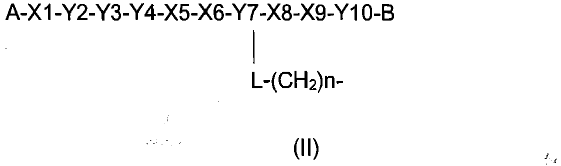
ist, worin n = 2;
A ausgewählt wird aus der Gruppe bestehend aus Ace-Asp, Ace-Asn und Ace-Pro;
X1 ausgewählt wird aus der Gruppe bestehend aus Glu, Aada, Arg, hArg und Leu;
Y2 Gln oder Glu ist;
Y3 Gln oder Glu ist;
Y4 Leu ist;
X5 ausgewählt wird aus der Gruppe bestehend aus His, 4Taz und 5Taz;
X6 ausgewählt wird aus der Gruppe bestehend aus Ser, Thr, Asn und aThr;
Y7 Gln oder Glu ist;
X8 ausgewählt wird aus der Gruppe bestehend aus Orn, Lys, Dap und Dab;
X9 ausgewählt wird aus der Gruppe bestehend aus Glu, Aada, Arg, hArg und Leu;
Y10 Lys oder Orn ist;
B Ile-Thr-Leu-NH2 ist;
L CO ist;
R2 und R7 CH3 sind;
R8 eine Formel Q-(CH2)s- aufweist, worin s = 2 ist und Q ausgewählt wird aus der Gruppe bestehend aus NH2CO-, CH3CONH-, HOOC- und CH3OOC-, oder R8 eine allgemeine Formel (III) aufweist: 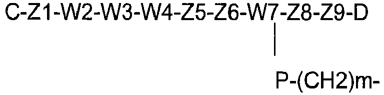
wobei m = 2 ist;
C Ace-Asp, Ace-Asn oder Ace-Pro ist;
Z1 ausgewählt wird aus der Gruppe bestehend aus Glu, Aada, Arg und hArg;
W2 Gln oder Glu ist;
W3 Gln oder Glu ist;
W4 Leu ist;
Z5 ausgewählt wird aus der Gruppe bestehend aus Ser, Gly, Ala und Aib;
Z6 ausgewählt wird aus der Gruppe bestehend aus Gln, Glu und Ser;
W7 Gln oder Glu ist;
Z8 ausgewählt wird aus der Gruppe bestehend aus Orn, Lys, Dap und Dab;
Z9 eine Aminosäure ausgewählt aus der Gruppe bestehend aus Glu, Aada, Arg und hArg ist;
D NH2 ist;
P CO ist;
R3, R4, R5, R6, unabhängig voneinander H oder Methyl sind.

2. Eine Verbindung gemäß Anspruch 1, worin:
die Stickstoffatome des Makrozyklus mit einem Metallion Me ausgewählt aus der Gruppe bestehend aus Fe, Mn und Ru in jeder erlaubten Oxidationsstufe komplexiert sind;
R1 die allgemeine Formel (II) aufweist, wobei:
A Ace-Asp ist;
X1 Glu oder Arg oder Leu ist;
Y2 Gln oder Glu ist;
Y3 Gln oder Glu ist;
Y4 Leu ist;
X5 His ist;
X6 ausgewählt wird aus der Gruppe bestehend aus Ser, Thr, Asn und aThr;
Y7 Gln oder Glu ist;
X8 Lys ist;
X9 Glu oder Arg oder Leu ist;
Y10 Lys oder Orn ist;
B Ile-Thr-Leu-NH2 ist;
L CO ist;
R2 und R7 CH3 sind;
R8 eine Formel Q-(CH2)s- aufweist, worin s = 2 ist und Q ausgewählt wird aus der Gruppe bestehend aus NH2CO-, CH3CONH-, HOOC- und CH3OOC-, oder R8 eine allgemeine Formel (III) aufweist, worin:
C Ace-Asp ist;
Z1 Glu oder Arg ist;
W2 Gln oder Glu ist;
W3 Gln oder Glu ist;
W4 Leu ist;
Z5 ausgewählt wird aus der Gruppe bestehend aus Ser, Gly, Ala und Aib;
Z6 ausgewählt wird aus der Gruppe bestehend aus Gln, Glu und Ser;
W7 Gln oder Glu ist;
Z8 Lys ist;
Z9 Glu oder Arg ist;
D NH2 ist;
P CO ist;
R3, R4, R5, R6, unabhängig voneinander H oder Methyl sind.

3. Eine Verbindung gemäß den Ansprüchen 1-2, die kovalent an ein Antigen; einen Antikörper oder dessen Fragment; ein Enzym gebunden ist.

4. Eine Verbindung gemäß den Ansprüchen 1-3, die von einer festen Matrix; Nanopartikeln oder Elektroden getragen wird.

5. Die Verwendung einer Verbindung gemäß den Ansprüchen 1-4 als Katalysator in den folgenden Reaktionen: Epoxidierung, Oxidation, Hydroxylierung, Nitrierung oder Peroxidation.

6. Die Verwendung einer Verbindung gemäß den Ansprüchen 1-4 in:
Immunodiagnostika-Kit; Immunhistochemie; *in-situ*-Hybridisierungskit; ELISA;
Southern Blotting; Northern Blotting; Western Blotting; oder in Zytofluorometrie.

7. Die Verwendung einer Verbindung gemäß den Ansprüchen 1-4
- zum Abbau von Schadstoffen.

## Revendications

1. Composé de formule générale (I) 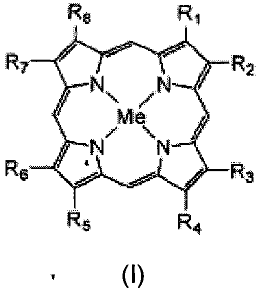 dans laquelle :
les atomes d'azote du macrocycle contenant de l'azote sont en complexe avec un ion métallique Me, choisi dans le groupe constitué par Fe, Mn, Ru, en tout état d'oxydation permis ;
R¹ est un groupe de formule générale (II) : 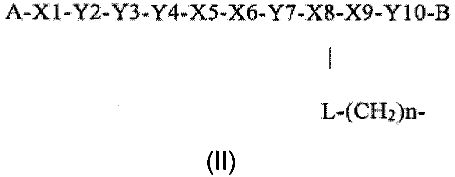 dans laquelle n = 2 ;
A est choisi dans le groupe constitué par Ace-Asp, Ace-Asn et Ace-Pro ;
X1 est choisi dans le groupe constitué par Glu, Aada, Arg, hArg, et Leu ;
Y2 est Gln ou Glu ;
Y3 est Gln ou Glu ;
Y4 est Leu ;
X5 est choisi dans le groupe constitué par His, 4Taz et 5Taz ;
X6 est choisi dans le groupe constitué par Ser, Thr, Asn et aThr ;
Y7 est Gln ou Glu ;
X8 est choisi dans le groupe constitué par Orn, Lys, Dap et Dab ;
X9 est choisi dans le groupe constitué par Glu, Aada, Arg, hArg, et Leu ;
Y10 est Lys ou Orn ;
B est Ile-Thr-Leu-NH2 ;
L est CO ;
R2 et R7 sont CH3 ;
R8 a la formule Q-(CH2)s- dans laquelle s = 2 et Q est choisi dans le groupe constitué par NH2CO-, CH3CONH-, HOOC- et CH3OOC-, ou R8 a la formule générale (III) : 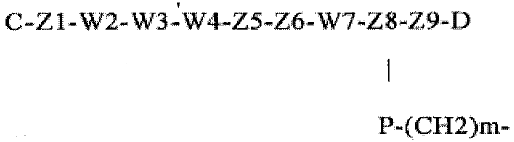 dans laquelle m = 2 ;
C est Ace-Asp, Ace-Asn ou Ace-Pro ;
Z1 est choisi dans le groupe constitué par Glu, Aada, Arg et hArg ;
W2 est Gln ou Glu ;
W3 est Gln ou Glu ;
W4 est Leu ;
Z5 est choisi dans le groupe constitué par Ser, Gly, Ala et Aib ;
Z6 est choisi dans le groupe constitué par Gln, Glu et Ser ;
W7 est Gln ou Glu ;
Z8 est choisi dans le groupe constitué par Orn, Lys, Dap et Dab ;
Z9 est un acide aminé choisi dans le groupe constitué par Glu, Aada, Arg et hArg ;
D est NH2 ;
P est CO ;
R3, R4, R5, R6, indépendamment les uns des autres sont H ou le méthyle,

2. Composé selon la revendication 1, dans lequel :
les atomes d'azote du macrocycle sont en complexe avec un ion métallique Me, choisi dans le groupe constitué par Fe, Mn et Ru, en tout état d'oxydation permis ;
R1 est de formule générale (II), dans laquelle :
A est Ace-Asp ;
X1 est Glu ou Arg ou Leu ;
Y2 est Gln ou Glu ;
Y3 est Gln ou Glu ;
Y4 est Leu ;
X5 est His ;
X6 est choisi dans le groupe constitué par Ser, Thr, Asn et aThr ;
Y7 est Gln ou Glu ;
X8 est Lys ;
X9 est Glu ou Arg, ou Leu ;
Y10 est Lys ou Orn ;
B est Ile-Thr-Leu-NH2 ;
L est CO ;
R2 et R7 sont CH3 ;
R8 a la formule Q-(CH2)s- dans laquelle s = 2 et Q est choisi dans le groupe constitué par NH2CO-, CH3CONH-, HOOC- et CH3OOC-, ou R8 a la formule générale (III), dans laquelle :
C est Ace-Asp ;
Z1 est Glu ou Arg ;
W2 est Gln ou Glu ;
W3 est Gln ou Glu ;
W4 est Leu ;
Z5 est choisi dans le groupe constitué par Ser, Gly, Ala et Aib ;
Z6 est choisi dans le groupe constitué par Gln, Glu et Ser ;
W7 est Gln ou Glu ;
Z8 est Lys ;
Z9 est Glu ou Arg ;
DestNH2;
P est CO ;
R3, R4, R5, R6, indépendamment les uns des autres, sont H ou le méthyle.

3. Composé selon les revendications 1-2, qui est lié par covalence à un antigène ; un anticorps ou un fragment de celui-ci ; une enzyme.

4. Composé selon les revendications 1-3, qui est supporté sur une matrice solide ; des nanoparticules ou une électrode.

5. Utilisation d'un composé selon les revendications 1-4 en tant que catalyseur dans les réactions suivantes : époxydation, oxydation, hydroxylation, nitration ou peroxydation.

6. Utilisation d'un composé selon les revendications 1-4 dans : kit d'immunodiagnostic ; immunohistochimie ; trousse d'hybridation in situ ; ELISA ; Southern blot ; Northern blot ; Western blot ; ou en cytofluorimétrie.

7. Utilisation d'un composé selon les revendications 1-4 :
- pour la dégradation de polluants.


### SEQUENCE LISTING

<110> Pavone, Vincenzo
   Nastri, Flavia
   Maglio, Ornella
   Lombardi, Angelina
<120> HIGH-EFFICIENCY CATALYSTS, PREPARATION AND USE THEREOF
<130> 9208MEUR
<160> 14
<170> PatentIn version 3.5
<210> 1
   <211> 12
   <212> PRT
   <213> Unknown
<220>
   <223> synthetic peptide
<220>
   <221> misc_feature
   <222> (1)..(1)
   <223> nothing; or acetyl-Asp; acetyl-Asn; acetyl-Pro; acetyl-Lys-Pro; acetyl-ornitine-Pro
<220>
   <221> misc_feature
   <222> (2)..(2)
   <223> [optionally succinylated or acetylated] [Glu; alpha-aminoadipic acid; Arg; homoArg; Leu; Lys; Orn]
<220>
   <221> misc_feature
   <222> (3)..(3)
   <223> Gln, Glu, Lys, Orn
<220>
   <221> misc_feature
   <222> (4)..(4)
   <223> Gln, Glu, Lys, Orn
<220>
   <221> misc_feature
   <222> (6)..(6)
   <223> His, homoCys, Met, beta(4-thiazolyl)-Ala, beta(5-thiazolyl)-Ala
<220>
   <221> misc_feature
   <222> (7)..(7)
   <223> Ser, Thr, Asn, Gln, alloThr, Glu, Lys, Orn
<220>
   <221> misc_feature
   <222> (8)..(8)
   <223> Gln, Glu, Lys, Orn
<220>
   <221> misc_feature
   <222> (9)..(9)
   <223> -(CH2)n-CO-[delta-Orn; epsilon-Lys; beta-2,3-diaminopropionic acid; gamma-2,4-diaminobutyric acid] or -(CH2)n-NH-[gamma-Glu; delta alpha-amino adipic acid]
<220>
   <221> misc_feature
   <222> (10)..(10)
   <223> Glu; alpha-aminoadipic acid; Arg; homoArg; Leu; Lys; Orn
<220>
   <221> misc_feature
   <222> (11)..(11)
   <223> Gln; Glu; Lys; Orn
<220>
   <221> misc_feature
   <222> (12)..(12)
   <223> -NH2; -Ile-NH2; -Ile-Thr-NH2; -Ile-Thr-Leu-NH2; -Ile-Thr-Leu-Lys-NH2; -Ile-Thr-Leu-Orn-NH2
<400> 1
   Xaa Xaa Xaa Xaa Leu Xaa Xaa Xaa Xaa Xaa Xaa Xaa
   1               5                   10
<210> 2
   <211> 11
   <212> PRT
   <213> Unknown
<220>
   <223> synthetic peptide
<220>
   <221> misc_feature
   <222> (1)..(1)
   <223> nothing; or acetyl-Asp; acetyl-Asn; acetyl-Pro; acetyl-LysPro; acetyl-OrnPro
<220>
   <221> misc_feature
   <222> (2)..(2)
   <223> [optionally succinylated or acetylated] [Glu; alpha-aminoadipic acid; Arg; homoArg; Leu; Lys; Orn;]
<220>
   <221> misc_feature
   <222> (3)..(3)
   <223> Gln; Glu; Lys; Orn;
<220>
   <221> misc_feature
   <222> (4)..(4)
   <223> Gln; Glu; Lys; Orn;
<220>
   <221> misc_feature
   <222> (6)..(6)
   <223> Ser; Gly; Ala; alpha-amino isobutyric acid;
<220>
   <221> misc_feature
   <222> (7)..(7)
   <223> Gln; Glu; Lys; Orn; Ser; Thr; Asn; allo-Thr
<220>
   <221> misc_feature
   <222> (8)..(8)
   <223> Gln; Glu; Lys; Orn;
<220>
   <221> misc_feature
   <222> (9)..(9)
   <223> -(CH2)m-CO-[epsilon-Lys; beta-2,3-diaminopropionic acid; gamma-2,4-diaminobutyrric acid; delta-Orn] or -(CH2)m-NH-[gamma-Glu; delta alpha-amino adipic acid]
<220>
   <221> misc_feature
   <222> (10)..(10)
   <223> Glu; alpha-aminoadipic acid; Orn; Lys; Arg; homoArg; Leu
<220>
   <221> misc_feature
   <222> (11)..(11)
   <223> -NH2; Lys-NH2; Orn-NH2
<400> 2
   Xaa Xaa Xaa Xaa Leu Xaa Xaa Xaa Xaa Xaa Xaa
   1               5                   10
<210> 3
   <211> 14
   <212> PRT
   <213> Unknown
<220>
   <223> synthetic peptide
<220>
   <221> misc_feature
   <222> (1)..(1)
   <223> acetyl-Asp
<220>
   <221> misc_feature
   <222> (9)..(9)
   <223> -CH2-CH2-CO-N-epsilon-Lys-
<220>
   <221> misc_feature
   <222> (14)..(14)
   <223> Leu-NH2
<400> 3
   Xaa Glu Gln Gln Leu His Ser Gln Xaa Arg Lys Ile Thr Xaa
   1               5                   10
<210> 4
   <211> 10
   <212> PRT
   <213> Unknown
<220>
   <223> synthetic peptide
<220>
   <221> misc_feature
   <222> (1)..(1)
   <223> acetyl-Asp;
<220>
   <221> misc_feature
   <222> (9)..(9)
   <223> -CH2-CH2-CO-N-epsilon-Lys
<220>
   <221> misc_feature
   <222> (10)..(10)
   <223> Arg-NH2
<400> 4
   Xaa Glu Gln Gln Leu Ser Ser Gln Xaa Xaa
   1               5                   10
<210> 5
   <211> 14
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsylon-Lys-
<220>
   <221> MISC_FEATURE
   <222> (14)..(14)
   <223> Leu-NH2
<400> 5
   Xaa Glu Gln Gln Leu His Ser Gln Xaa Leu Lys Ile Thr Xaa
   1               5                   10
<210> 6
   <211> 10
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsilon-Lys-
<220>
   <221> MISC_FEATURE
   <222> (10)..(10)
   <223> Arg-NH2
<400> 6
   Xaa Glu Gln Gln Leu Ser Ser Gln Xaa Xaa
   1               5                   10
<210> 7
   <211> 14
   <212> PRT
   <213> Unknown
<220>
   <223> synthetic peptide
<220>
   <221> misc_feature
   <222> (1)..(1)
   <223> acetyl-Asp
<220>
   <221> misc_feature
   <222> (9)..(9)
   <223> -CH2-CH2-CO-N-epsilon-Lys
<220>
   <221> misc_feature
   <222> (14)..(14)
   <223> Leu-NH2
<400> 7
   Xaa Leu Gln Gln Leu His Ser Gln Xaa Arg Lys Ile Thr Xaa
   1               5                   10
<210> 8
   <211> 14
   <212> PRT
   <213> Unknown
<220>
   <223> synthetic peptide
<220>
   <221> misc_feature
   <222> (1)..(1)
   <223> acetyl-Asp
<220>
   <221> misc_feature
   <222> (9)..(9)
   <223> -CH2-CH2-CO-N-epsilon-Lys
<220>
   <221> misc_feature
   <222> (14)..(14)
   <223> Leu-NH2
<400> 8
   Xaa Glu Gln Gln Leu His Ser Gln Xaa Arg Lys Ile Thr Xaa
   1               5                   10
<210> 9
   <211> 14
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsilon-Lys-
<220>
   <221> MISC_FEATURE
   <222> (14)..(14)
   <223> Leu-NH2
<400> 9
   Xaa Glu Gln Gln Leu His Ser Gln Xaa Arg Lys Ile Thr Xaa
   1               5                   10
<210> 10
   <211> 10
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsilon-Lys-
<220>
   <221> MISC_FEATURE
   <222> (10)..(10)
   <223> Arg-NH2
<400> 10
   Xaa Glu Gln Gln Leu Gly Ser Gln Xaa Xaa
   1               5                   10
<210> 11
   <211> 14
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsilon-Lys-
<220>
   <221> MISC_FEATURE
   <222> (14)..(14)
   <223> Leu-NH2
<400> 11
   Xaa Glu Gln Gln Leu His Ser Gln Xaa Arg Lys Ile Thr Xaa
   1               5                   10
<210> 12
   <211> 10
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsilon-Lys-
<220>
   <221> MISC_FEATURE
   <222> (10)..(10)
   <223> Arg-NH2
<400> 12
   Xaa Leu Gln Gln Leu Ser Ser Gln Xaa Xaa
   1               5                   10
<210> 13
   <211> 14
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-epsilon Lys-
<220>
   <221> MISC_FEATURE
   <222> (14)..(14)
   <223> Leu-NH2
<400> 13
   Xaa Glu Gln Gln Leu His Ser Gln Xaa Arg Lys Ile Thr Xaa
   1               5                   10
<210> 14
   <211> 10
   <212> PRT
   <213> Unknown
<220>
   <223> synthetic peptide
<220>
   <221> MISC_FEATURE
   <222> (1)..(1)
   <223> acetyl-Asp
<220>
   <221> MISC_FEATURE
   <222> (9)..(9)
   <223> -CH2-CH2-CO-N-Lys-
<220>
   <221> MISC_FEATURE
   <222> (10)..(10)
   <223> Leu-NH2
<400> 14
   Xaa Glu Gln Gln Leu Ser Ser Gln Xaa Xaa
   1               5                   10